# EUROPEAN PATENT SPECIFICATION

(11) **EP 3 436 605 B1**
(45) Date of publication and mention of the grant of the patent: **18.06.2025**
(21) Application number: 17772883.9
(22) Date of filing: 31.03.2017
(51) Int. Cl.: C12Q 1/68, C12Q 1/6844, C12Q 1/686

(54) **AMPLIFICATION OF TARGET SEQUENCES**
AMPLIFIKATION VON ZIELSEQUENZEN
AMPLIFICATION DE SÉQUENCES CIBLES

(30) Priority: 31.03.2016 AU 2016901197
(43) Date of publication of application: 06.02.2019
(73) Proprietor: Revvity Holdings, Inc., Waltham, MA 02451 (US)
(72) Inventor: JASPER, Melinda, Collinswood, South Australia 5081 (AU); FRASER, Michelle, Torrens Park, South Australia 5062 (AU)
(74) Representative: Marks & Clerk LLP
(86) International application number: PCT/AU2017/050281
(87) International publication number: WO 2017/165925

(56) References cited:
- EP-A1- 2 660 331
- WO-A1-2009/105531
- WO-A1-2011/131192
- US-A- 5 994 058
- US-A1- 2010 184 152
- US-A1- 2011 033 862
- US-B2- 9 249 459
- TELENIUS H ET AL: "Degenerate oligonucleotide-primed PCR: General amplification of target DNA by a single degenerate primer", GENOMICS, ACADEMIC PRESS, SAN DIEGO, US, vol. 13, no. 3, 1 July 1992 (1992-07-01), pages 718 - 725, XP026059976, ISSN: 0888-7543, [retrieved on 19920701], DOI: 10.1016/0888-7543(92)90147-K
- NAKAYAMA, T. ET AL.: "Isolation of the 5'-flanking region of genes by thermal asymmetric interlaced polymerase chain reaction", MEDICAL SCIENCE MONITOR., vol. 7, 2001, pages 345 - 349, XP055426564
- WANG, Z ET AL.: "Fusion primer and nested integrated PCR (FPNI-PCR): a new high- efficiency strategy for rapid chromosome walking or flanking sequence cloning", BMC BIOTECHNOLOGY, vol. 11, 2011, pages 109, XP055015224

## Description

### FIELD

The present disclosure relates to methods, kits and products for amplifying target DNA sequences from genomic DNA.

### BACKGROUND

The ability to amplify specific target sequences present in genomic DNA is used in a variety of fields, such as for research purposes, recombinant DNA technology, genetic screening, and for forensic analyses.

Whilst target specific amplification is extensively used, it carries a number of drawbacks, particularly under circumstances where the amount of starting genomic material is limited. For example, target specific amplification often leads to rapid depletion of limited genomic material and typically only allows for the analysis of specifically amplified sequences. As a consequence, it is often difficult to obtain other important genetic information from the remainder of the genomic DNA.

Whole genome amplification (WGA) is a process that was in part developed to address the problem of having limited genomic DNA for analysis. Whole genome amplification allows amplification of an entire genome using only very small amounts of starting material, and as such can provide immortalization of a limited sample so that other desired analyses on the sample can be undertaken.

A variety of methods have been developed for whole genome amplification, including Degenerate Oligonucleotide PCR (DOP-PCR), Primer Extension Preamplification (PEP) and Multiple Displacement Amplification (MDA). DOP-PCR and PEP are based on standard PCR techniques, while MDA is a non-PCR amplification technique utilising the strand displacement ability of some polymerases. The genomic DNA amplified by whole genome amplification may then be analysed, for example by techniques such as comparative genomic hybridization (CGH) analysis, SNP analysis, and sequencing.

US9249459 discloses a method for analyzing DNA from a single cell, said method comprising: (a) performing WGA of the genome using degenerate primers (DOP-PCR) (b) preamplifying the amplified genome using a plurality of target-specific primer pairs to produce a pre-amplification reaction mixture comprising a plurality of amplicons specific for a plurality of target nucleic acids

However, whilst whole genome amplification provides the ability to amplify genomic DNA for analysis, the amplification of specific target sequences from the amplified genomic DNA carries with it a number of disadvantages, such as one or more of allele dropout and/or bias issues introduced during the WGA process, the possibility of uneven sequence coverage, over or under representation of specific regions, reproducibility, and the introduction of errors into the specific target sequences.

Accordingly, there is a need to provide improved methods and/or products for amplifying specific target sequences from whole genome amplified DNA.

### SUMMARY

The present disclosure relates to methods, and kits for amplifying target DNA sequences from genomic DNA.

Certain embodiments of the present disclosure provide a method of amplifying a target DNA sequence from genomic DNA, the method comprising amplifying the genomic DNA with one or more degenerate oligonucleotide primers in the presence of one or more target DNA sequence specific primers, thereby amplifying the target DNA sequence.

Certain embodiments of the present disclosure provide a method of amplifying a target DNA sequence from genomic DNA, the method comprising amplifying the target DNA sequence with one or more target DNA sequence specific primers for at least one or more cycles of amplification of genomic DNA with one or more degenerate oligonucleotide primers, thereby amplifying the target DNA sequence.

Certain embodiments of the present disclosure provide a method of amplifying a target DNA sequence from genomic DNA, the method comprising:
amplifying genomic DNA with one or more degenerate oligonucleotide primers; and
for at least one or more cycles of the amplification of the genomic DNA also amplifying the target DNA sequence with one or more target DNA sequence specific primers,
thereby amplifying the target DNA sequence.

Certain embodiments of the present disclosure provide a method of amplifying a target DNA sequence from genomic DNA, the method comprising:
performing whole genome amplification of the genomic DNA with one or more degenerate oligonucleotide primers; and
for at least part of the whole genome amplification also amplifying the target DNA sequence with one or more target DNA sequence specific primers,
thereby amplifying the target DNA sequence.

Certain embodiments of the present disclosure provide a target DNA sequence amplified by a method as described herein.

Certain embodiments of the present disclosure provide a method of assessing nucleotide content of a target DNA sequence amplified from genomic DNA by a method as described herein.

Certain embodiments of the present disclosure provide a method of assessing nucleotide content of a target DNA sequence in genomic DNA, the method comprising:
amplifying the genomic DNA with one or more degenerate oligonucleotide primers in the presence of one or more target DNA sequence specific primers to produce an amplified target DNA sequence; and
assessing the nucleotide content of the amplified target DNA sequence so as to assess the nucleotide content of the target DNA sequence.

Certain embodiments of the present disclosure provide a method of assessing nucleotide content of a target DNA sequence in genomic DNA, the method comprising amplifying the target DNA sequence with one or more target DNA sequence specific primers for at least one or more cycles of amplification of genomic DNA with one or more degenerate oligonucleotide primer to produce an amplified target DNA sequence, and assessing the nucleotide content of the amplified target DNA sequence.

Certain embodiments of the present disclosure provide a method of assessing nucleotide content of a target DNA sequence in genomic DNA, the method comprising:
amplifying genomic DNA with one or more degenerate oligonucleotide primers; and
for at least one or more cycles of the amplification of the genomic DNA also amplifying the target DNA sequence with one or more target DNA sequence specific primers to produce an amplified target DNA sequence; and
assessing the nucleotide content of the amplified target DNA sequence so as to assess the nucleotide content of the target DNA sequence.

Certain embodiments of the present disclosure provide a method of assessing nucleotide content of a target DNA sequence in genomic DNA, the method comprising:
performing whole genome amplification of the genomic DNA with one or more degenerate oligonucleotide primers;
for at least part of the whole genome amplification also amplifying the target DNA sequence with one or more target DNA sequence specific primers to produce an amplified target DNA sequence; and
assessing the nucleotide content of the amplified target DNA sequence so as to assess the nucleotide content of the target DNA sequence.

Certain embodiments of the present disclosure provide a method of determining the nucleotide sequence of a target DNA sequence in genomic DNA, the method comprising:
amplifying the genomic DNA with one or more degenerate oligonucleotide primers in the presence of one or more target DNA sequence specific primers to produce an amplified target DNA sequence; and
determining the nucleotide sequence of the amplified target DNA sequence,
thereby determining the nucleotide sequence of the target DNA sequence.

Certain embodiments of the present disclosure provide a method of pre-implantation genetic screening of an embryo, the method comprising:
amplifying the genomic DNA from the embryo with one or more degenerate oligonucleotide primers in the presence of one or more target DNA sequence specific primers to produce an amplified target DNA sequence; and
assessing the amplified target DNA sequence to screen the embryo.

Certain embodiments of the present disclosure provide a method of pre-implantation genetic screening of an embryo, the method comprising amplifying a target DNA sequence from the embryo with one or more target DNA sequence specific primers for at least one or more cycles of amplification of genomic DNA from the embryo with one or more degenerate oligonucleotide primers to produce an amplified target DNA sequence, and assessing the amplified target DNA sequence to screen the embryo.

Certain embodiments of the present disclosure provide a method of pre-implantation genetic screening of an embryo, the method comprising:
amplifying genomic DNA from the embryo with one or more degenerate oligonucleotide primers;
for at least one or more cycles of the amplification of the genomic DNA also amplifying a target DNA sequence from the embryo with one or more target DNA sequence specific primers to produce an amplified target DNA sequence; and
assessing the amplified target DNA sequence to screen the embryo.

Certain embodiments of the present disclosure provide a method of pre-implantation genetic screening of an embryo, the method comprising:
performing whole genome amplification of the genomic DNA from the embryo with one or more degenerate oligonucleotide primers;
for at least part of the whole genome amplification also amplifying a target DNA sequence from the embryo with one or more target DNA sequence specific primers to produce an amplified target DNA sequence; and
assessing the amplified target DNA sequence to screen the embryo.

Certain embodiments of the present disclosure provide a method of pre-implantation genetic screening of an oocyte, the method comprising:
amplifying the genomic DNA from an oocyte or a polar body thereof with one or more degenerate oligonucleotide primers in the presence of one or more target DNA sequence specific primers to produce an amplified target DNA sequence; and
assessing the amplified target DNA sequence to screen the oocyte.

Certain embodiments of the present disclosure provide a method of pre-implantation genetic screening of an oocyte, the method comprising amplifying a target DNA sequence from the oocyte or a polar body thereof with one or more target DNA sequence specific primers for at least one or more cycles of amplification of genomic DNA from the oocyte or polar body with one or more degenerate oligonucleotide primers to produce an amplified target DNA sequence, and assessing the amplified target DNA sequence to screen the oocyte.

Certain embodiments of the present disclosure provide a method of pre-implantation genetic screening of an oocyte, the method comprising:
amplifying genomic DNA from the oocyte or a polar body thereof with one or more degenerate oligonucleotide primers;
for at least one or more cycles of the amplification of the genomic DNA also amplifying a target DNA sequence from the oocyte or polar body with one or more target DNA sequence specific primers to produce an amplified target DNA sequence; and
assessing the amplified target DNA sequence to screen the oocyte.

Certain embodiments of the present disclosure provide a method of pre-implantation genetic screening of an oocyte, the method comprising:
performing whole genome amplification of the genomic DNA from the oocyte or a polar body thereof with one or more degenerate oligonucleotide primers;
for at least part of the whole genome amplification also amplifying a target DNA sequence from the oocyte or polar body with one or more target DNA sequence specific primers to produce an amplified target DNA sequence; and
assessing the amplified target DNA sequence to screen the oocyte.

Certain embodiments of the present disclosure provide a method of screening of sperm, the method comprising:
amplifying the genomic DNA from one or more sperm with one or more degenerate oligonucleotide primers in the presence of one or more target DNA sequence specific primers to produce an amplified target DNA sequence; and
assessing the amplified target DNA sequence to screen the sperm.

Certain embodiments of the present disclosure provide a method of screening of sperm, the method comprising amplifying a target DNA sequence from one or more sperm with one or more target DNA sequence specific primers for at least one or more cycles of amplification of genomic DNA from the one or more sperm with one or more degenerate oligonucleotide primers to produce an amplified target DNA sequence, and assessing the amplified target DNA sequence to screen the sperm.

Certain embodiments of the present disclosure provide a method of screening of sperm, the method comprising:
amplifying genomic DNA from one or more sperm with one or more degenerate oligonucleotide primers;
for at least one or more cycles of the amplification of the genomic DNA also amplifying a target DNA sequence from the one or more sperm with one or more target DNA sequence specific primers to produce an amplified target DNA sequence; and
assessing the amplified target DNA sequence to screen the sperm.

Certain embodiments of the present disclosure provide a method of screening of sperm, the method comprising:
performing whole genome amplification of the genomic DNA from one or more sperm with one or more degenerate oligonucleotide primers;
for at least part of the whole genome amplification also amplifying a target DNA sequence from the one or more sperm with one or more target DNA sequence specific primers to produce an amplified target DNA sequence; and
assessing the amplified target DNA sequence to screen the sperm.

Certain embodiments of the present disclosure provide a method of prenatal screening of a fetus, the method comprising:
amplifying genomic DNA from the fetus with one or more degenerate oligonucleotide primers in the presence of one or more target DNA sequence specific primers to produce an amplified target DNA sequence; and
assessing the amplified target DNA sequence to screen the fetus.

Certain embodiments of the present disclosure provide a method of pre-natal screening of a fetus, the method comprising amplifying a target DNA sequence from the fetus with one or more target DNA sequence specific primers for at least one or more cycles of amplification of genomic DNA from the fetus with one or more degenerate oligonucleotide primers to produce an amplified target DNA sequence, and assessing the amplified target DNA sequence to screen the fetus.

Certain embodiments of the present disclosure provide a method of pre-natal screening of a fetus, the method comprising:
amplifying genomic DNA from the fetus with one or more degenerate oligonucleotide primers;
for at least one or more cycles of the amplification of the genomic DNA also amplifying a target DNA sequence from the fetus with one or more target DNA sequence specific primers to produce an amplified target DNA sequence; and
assessing the amplified target DNA sequence to screen the fetus.

Certain embodiments of the present disclosure provide a method of pre-natal screening of a fetus, the method comprising:
performing whole genome amplification of the genomic DNA from one or more sperm with one or more degenerate oligonucleotide primers;
for at least part of the whole genome amplification also amplifying a target DNA sequence from the fetus with one or more target DNA sequence specific primers to produce an amplified target DNA sequence; and
assessing the amplified target DNA sequence to screen the fetus.

Certain embodiments of the present disclosure provide a kit for performing a method as described herein.

Certain embodiments of the present disclosure provide a use of a target DNA sequence amplified as described herein for preimplantation genetic screening, preimplantation genetic diagnosis, prenatal screening, screening for a disease, screening for a cancer, screening for a genetic marker, screening for a polymorphism, a mutation, a deletion, an insertion, a translocation, an expansion, an inversion, methylation status, and/or an epigenetic change, and/or forensic screening.

Certain embodiments of the present disclosure provide a combination product, the product comprises the following components:
(i) one or more degenerate oligonucleotide primers for whole genome amplification;
(ii) one or more primers for amplifying a target DNA sequence; and
(iii) one or more reagents and/or instructions for amplifying the target DNA sequence during whole genome amplification.

Other embodiments are described herein.

### BRIEF DESCRIPTION OF THE FIGURES

Certain embodiments are illustrated by the following figures. It is to be understood that the following description is for the purpose of describing particular embodiments only and is not intended to be limiting with respect to the description.

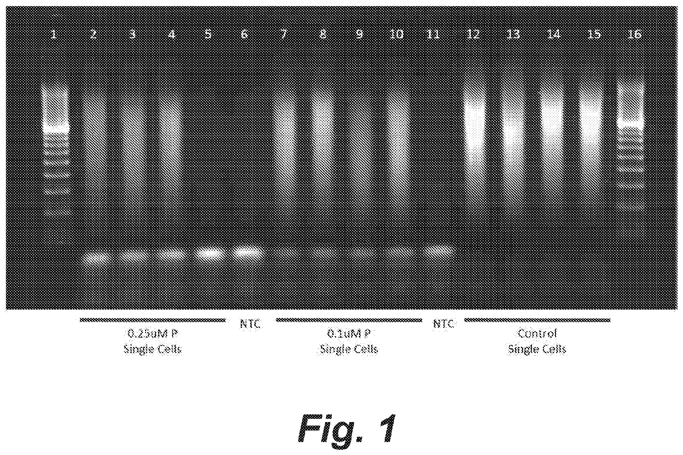

Figure 1 shows amplification products of single cells after whole genome amplification and gene targeting. Lanes 2-5 show single cell products as a result of whole genome amplification and gene targeting with a final primer concentration of 0.25uM. The cell in lane 5 failed amplification. Lanes 7-10 show single cell products as a result of whole genome amplification and gene targeting with a final primer concentration of 0.1uM. Weaker amplification is seen in cells amplified with gene targeting compared with the control cells (lanes 12-15).

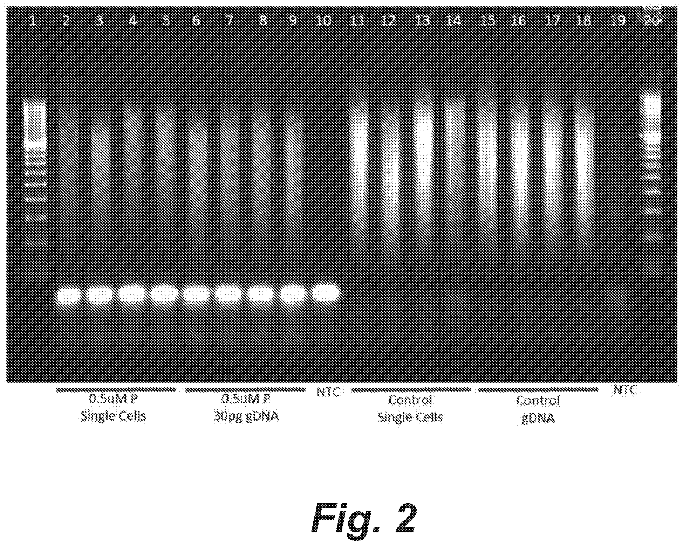

Figure 2 shows amplification products of single cells and 30pg gDNA after whole genome amplification. Lanes 2-9 show weaker amplification due to the presence of 0.5uM final concentration gene specific primers compared control samples in lanes 11-18.

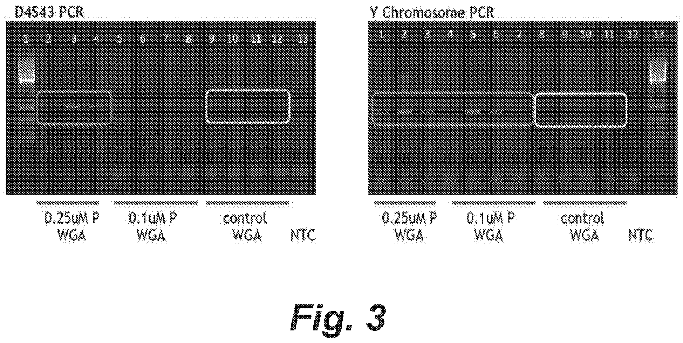

Figure 3 shows electrophoretogram of second round PCR generated amplicons after combined whole genome amplification and gene targeted single cell products were seeded into two gene specific PCRs. The D4S43 PCR gel shows enriched amplification of the target D4S43 marker due to the addition of 0.25uM gene specific primers (lanes 2-4) compared with the control (lanes 9-12). Similarly, the Y chromosome PCR shows enriched amplification of the targeted gene due to the addition of gene specific primers at final concentrations of 0.25uM and 0.1uM (lanes 1-3 and 4-7 respectively) compared with the control (lanes 8-11). Samples were electrophoresed with the molecular weight standard DMW-100M (Geneworks).

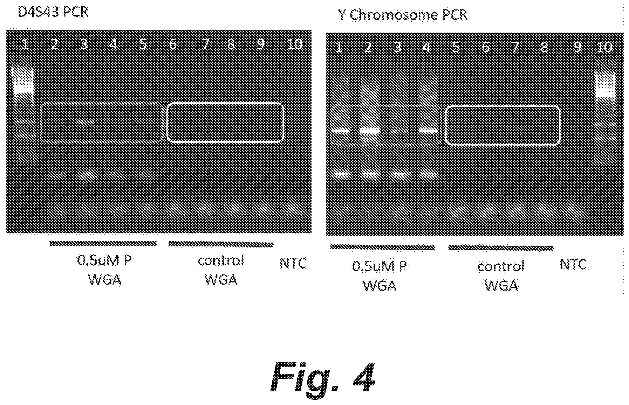

Figure 4 shows electrophoretogram of second round PCR generated amplicons after combined whole genome amplification and gene targeted single cell products were seeded into two gene specific PCRs. The D4S43 PCR gel shows enriched amplification of the target D4S43 marker due to the addition of 0.5uM gene specific primers (lanes 2-5) compared with the control (lanes 6-9). Similarly, the Y chromosome PCR shows enriched amplification of the targeted gene due to the addition of gene specific primers at a final concentration of 0.5uM (lanes 1-4) compared with the control lanes 5-8). Samples were electrophoresed with the molecular weight standard DMW-100M (Geneworks).

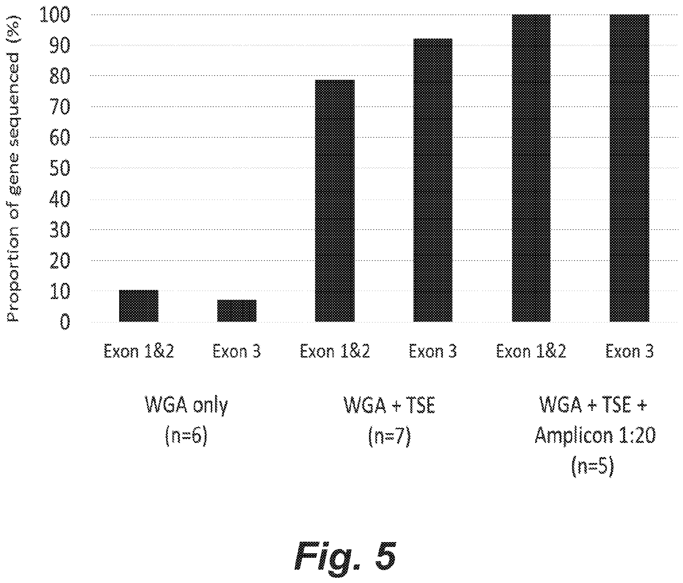

Figure 5 shows the breadth of coverage of HBB following combined whole genome amplification and gene targeted amplification compared to whole genome amplification only using next generation sequencing. After combined whole genome amplification and gene targeted amplification products were seeded into two gene specific PCRs. The second round PCR amplicons were pooled to generate a final dilution of 1:20 with the whole genome amplified DNA with gene targeted amplification. The whole genome amplification only PCR products (WGA), the combined whole genome amplification and gene targeted amplification products (WGA + TSE) and the 1:20 diluted pool were sequenced. The graph shows 100% breadth of coverage of both HBB targeted sequences (exon 1 and 2 and exon 3) was achieved when the 1:20 dilution of second round HBB PCR products were pooled back with the combined whole genome amplified samples with gene targeted amplification. Mean values plotted.

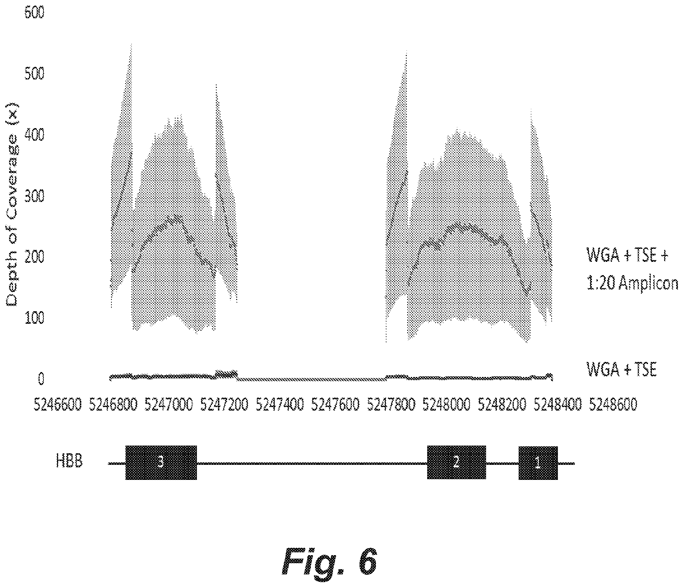

Figure 6 shows the depth of coverage for exon 1 and 2 and exon 3 of HBB following combined whole genome amplification and gene targeted amplification with further HBB PCR product pooling compared to whole genome amplification and gene targeted amplification using next generation sequencing. After combined whole genome amplification and gene targeted amplification products were seeded into two HBB specific PCRs. The second round HBB PCR amplicons were pooled to generate a final dilution of 1:20 with the whole genome amplified DNA with gene targeted amplification. The combined whole genome amplification and gene targeted amplification products (WGA + TSE) and the 1:20 diluted pooled DNA were sequenced. Mean depth of coverage of HBB sequence > 60x was achieved when the 1:20 diluted pooled DNA was sequenced.

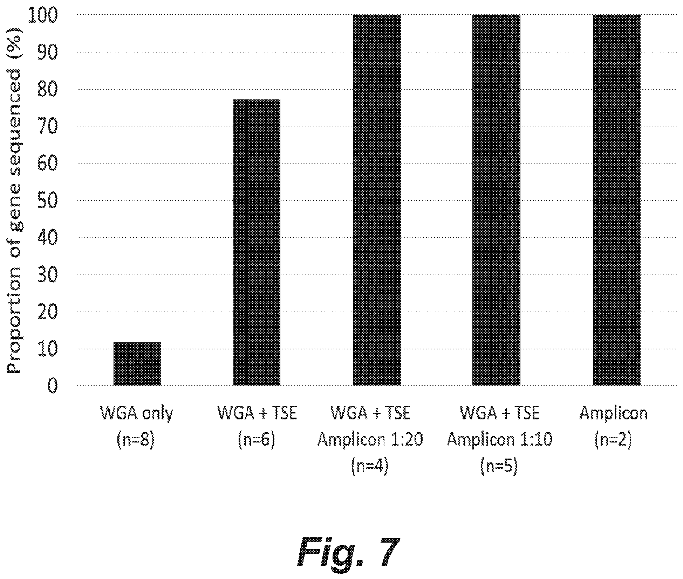

Figure 7 shows breadth of coverage of the targeted gene; HLA following combined whole genome amplification and gene targeted amplification compared to whole genome amplification only using next generation sequencing. After combined whole genome amplification and gene targeted amplification products were seeded into one HLA specific PCR. The second round HLA PCR amplicon was pooled to generate a final dilution of 1:20 and 1:10 with the whole genome amplified DNA with gene targeted amplification. The whole genome amplification only PCR products (WGA), the combined whole genome amplification and gene targeted amplification products (WGA + TSE), the 1:20 and 1:10 diluted pooled DNA and the second round HLA PCR amplicon (amplicon) were sequenced. The graph shows 100% breadth of coverage of HLA was achieved when the 1:20 and 1:10 dilution of second round HLA PCR product was pooled back with the combined whole genome amplified samples with gene targeted amplification and HLA PCR product alone. Mean values plotted.

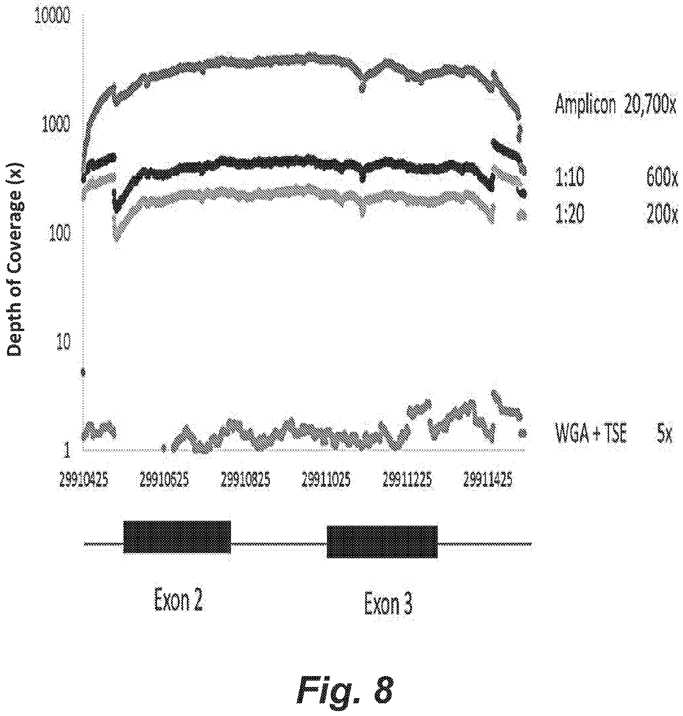

Figure 8 shows the depth of coverage for HLA following combined whole genome amplification and gene targeted amplification using next generation sequencing. After combined whole genome amplification and gene targeted amplification products were seeded into a HLA specific PCR. The second round HLA PCR amplicon was pooled to generate a final dilution of 1:20 and 1:10 with the whole genome amplified DNA with gene targeted amplification. The combined whole genome amplification and gene targeted amplification products (WGA + TSE), the 1:20 and 1:10 diluted pooled DNA and the second round HLA PCR amplicon (amplicon) were sequenced. Mean depth of coverage of HLA > 60x was achieved when the 1:20 and 1:10 diluted pooled DNA and the second round HLA PCR amplicon were sequenced.

### DETAILED DESCRIPTION

The present disclosure relates to methods, kits and products for amplifying target DNA sequences from genomic DNA.

The present disclosure is based on the determination that is possible to amplify specific target DNA sequences during whole genome amplification.

Certain embodiments of the present disclosure are directed to methods and products that have one or more combinations of advantages. For example, some of the advantages of some of the embodiments disclosed herein include one or more of the following: a reduction in the possibility of allele drop out when amplifying a target DNA sequence from whole genome amplified DNA; improved ability to analyse a target DNA sequence amplified from whole genome amplified DNA; reducing bias associated with amplification of a target DNA sequence amplified from whole genome amplified DNA; reducing the number of PCR cycles for amplifying a target DNA sequence from whole genome amplified DNA; an improvement in the time to amplify a specific target DNA sequence from whole genome amplified DNA; reduced use of consumables and/or human resources; reduced costs; and/or to provide a commercial alternative.

Certain embodiments of the present disclosure provide a method of amplifying a target DNA sequence from genomic DNA.

The term "amplifying", or variants such as "amplification" and "amplified", refers to the process of copying a nucleic acid to produce further copies of all or part of the nucleic acid. For example, amplification of a nucleic acid may be achieved enzymatically using a polymerase chain reaction (PCR), an isothermal method such as multiple displacement amplification using a suitable polymerase or rolling circle amplification. Other types of methods of amplification are contemplated. Methods for performing amplification are described herein.

The term "genomic DNA" refers to all or part of DNA derived from an organism, and includes chromosomal DNA, cellular DNA, mitochondrial DNA, and viral DNA. The genomic DNA may, for example, be isolated, processed or extracted from prokaryotic or eukaryotic cellular DNA, viral DNA, somatic cell DNA, germ cell DNA, gamete DNA, polar body DNA, embryonic cell DNA, or fetal DNA. The term includes DNA derived from exonic DNA, intronic DNA, non-coding DNA, RNA-coding DNA, non-repetitive DNA, repetitive DNA, transposon DNA, extrachromosomal DNA, organelle DNA, mitochondrial DNA, chloroplast DNA, viral DNA, plasmid DNA, nuclear DNA, extranuclear DNA, cytoplasmic DNA, cDNA and cell-free DNA. Other types of DNA are contemplated.

The term "target DNA sequence" refers to a specific region or part of DNA that is to be amplified. For example, the target DNA sequence may be all or part of a gene, a locus, a coding region, one or more exons and/or one or more introns, a SNP, a region to be assessed for a mutation, an allelic region, or all or part of a DNA coding for a RNA. Other types of target DNA sequences are contemplated.

Certain embodiments of the present disclosure provide a method of amplifying a target DNA sequence from genomic DNA, the method comprising amplifying the genomic DNA with one or more degenerate oligonucleotide primers in the presence of one or more target DNA sequence specific primers, thereby amplifying the target DNA sequence.

In certain embodiments, the amplifying comprises a polymerase chain reaction. Methods for using a polymerase chain reaction are as described herein. Methods for performing amplification are described, for example, in Fakruddin *et al.* (2013) *J Pharm Bioallied Sci* 5(4): 245-252.

In certain embodiments, the amplifying comprises an isothermal amplification reaction. Methods for performing isothermal amplification are known in the art, such as loop-mediated isothermal amplification (LAMP), strand displacement amplification (SDA), helicase-dependent amplification (HDA), and nicking enzyme amplification reaction (NEAR). In certain embodiments, the amplifying comprises a reaction using Multiple Displacement Amplification. Methods for performing Multiple Displacement Amplification WGA are known in the art, for example as described in Dean et al. (2002) Proc Natl. Acad. Sci USA 99: 5261. In certain embodiments, the amplifying comprises a reaction using rolling circle amplification. Other types of amplification are contemplated. Methods for amplifying are as described, for example, in Walker et al. (1992) Nucleic Acids Res. 20:1691-1696, Walker et al. (1993) PCR Methods Appl. 3:1-6, Notomi et al. (2000) Nucleic Acids Res. 28:e63, Tomita N et al. (2008) Nat. Protoc. 3:877-882, Lizardi et al. (1998) Nat. Genet. 19:225-232, Blanco et al. (1989) J. Biol. Chem. 264:8935-8940, and Dean et al. (2001) Genome Res. 11:1095-1099, and Fakruddin et al. (2013) J Pharm Bioallied Sci 5(4): 245-252.

In certain embodiments the target DNA sequence comprises all or part of a gene, a locus, a coding region, one or more exons and/or one or more introns, a region to be assessed for a mutation, an allelic region, a region having a SNP or other type of polymorphism, or all or part of a DNA coding for a RNA, a region to be assessed for the presence of absence of a mutation, a marker DNA, and/or a region used for identifying the presence or type of a DNA. Other types of target DNA sequences are contemplated.

In certain embodiments, the genomic DNA comprises DNA from one or more embryonic cells, DNA from an oocyte or a polar body thereof, DNA from sperm, DNA from one or more germ cells, DNA from one or more somatic cells, DNA from one or more human or animal cells, DNA from one or more plant cells, DNA from one or more cells from a microorganism, DNA from one or more cells for screening for a disease, DNA from one or more cancerous cells, DNA for forensic testing, mitochondrial DNA, extra chromosomal DNA, cell-free DNA, DNA from a sample comprising one or more cells, DNA from a sample comprising cell-free DNA, DNA from a cell lysate, DNA from a biopsy, DNA from a tissue sample, DNA from a cell sample, DNA from a bodily fluid, DNA from a blood sample, fetal DNA, cell-free DNA, DNA from amniotic fluid, DNA from a formalin fixed sample, DNA from a paraffin embedded sample, and DNA from a swab. Other types of DNA are contemplated. Methods for obtaining or using DNA in an amplification reaction are known in the art.

In certain embodiments, the genomic DNA comprises DNA from a lysed cell(s). In certain embodiments, the genomic DNA comprises non-purified DNA. In certain embodiments, the genomic DNA comprises extracted DNA. In certain embodiments, the genomic DNA comprises processed DNA. In certain embodiments, the genomic DNA comprises purified or partially purified DNA. In certain embodiments, the genomic DNA comprises cell-free DNA.

In certain embodiments, the method comprises an amount of genomic DNA to be amplified of less than 0. 5 pg, less than 1 pg, less than 2.5 pg, less than 5 pg, less than 10 pg, less than 25 pg, less than 50 pg, less than 100 pg, less than 250 pg, less than 500 pg, less than 1 ng, less than 2.5 ng, less than 5 ng or less than 10 ng. Other amounts are contemplated.

In certain embodiments, the method comprises an amount of genomic DNA to be amplified of at least 0. 5 pg, at least 1 pg, at least 2.5 pg, at least 5 pg, at least 10 pg, at least 25 pg, at least 50 pg, at least 100 pg, at least 250 pg, at least 500 pg, at least 1 ng, at least 2.5 ng, at least 5 ng or at least 10 ng. Other amounts are contemplated.

In certain embodiments, the method comprises an amount of genomic DNA to be amplified of 0.5 pg to 10 ng, 1 pg to 10 ng, 2.5 pg to 10 ng, 5 pg to 10 ng, 10 pg to 10 ng, 25 pg to 10 ng, 50 pg to 10 ng, 100 pg to 10 ng, 250 pg to 10 ng, 500 pg to 10 ng, 1 ng to 10 ng, 2.5 ng to 10 ng, 5 ng to 10 ng, 0.5 pg to 5 ng, 1 pg to 5 ng, 2.5 pg to 5 ng, 5 pg to 5 ng, 10 pg to 5 ng, 25 pg to 5 ng, 50 pg to 5 ng, 100 pg to 5 ng, 250 pg to 5 ng, 500 pg to 5 ng, 1 ng to 5 ng, 2.5 ng to 5 ng, 0.5 pg to2.5 ng, 1 pg to 2.5 ng, 2.5 pg to 2.5 ng, 5 pg to 2.5 ng, 10 pg to 2.5 ng, 25 pg to 2.5 ng, 50 pg to 2.5 ng, 100 pg to 2.5 ng, 250 pg to 2.5 ng, 500 pg to 2.5 ng, 1 ng to 2.5 ng, 0.5 pg to 1 ng, 1 pg to 1 ng, 2.5 pg to 1 ng, 5 pg to 1 ng, 10 pg to 1 ng, 25 pg to 1 ng, 50 pg to 1 ng, 100 pg to 1 ng, 250 pg to 1 ng, 500 pg to 1 ng, 0.5 pg to 500 pg, 1 pg to 500 pg, 2.5 pg to 500 pg, 5 pg to 500 pg, 10 pg to 500 pg, 25 pg to 500 pg, 50 pg to 500 pg, 100 pg to 500 pg, 250 pg to 500 pg, 0.5 pg to 250 pg, 1 pg to250 pg, 2.5 pg to 250 pg, 5 pg to 250 pg, 10 pg to250 pg, 25 pg to 250 pg, 50 pg to 250 pg, 100 pg to 250 pg, 0.5 pg to 100 pg, 1 pg to 100 pg, 2.5 pg to 100 pg, 5 pg to 100 pg, 10 pg to 100 pg, 25 pg to 100 pg, 50 pg to 100 pg, 0.5 pg to 50 pg, 1 pg to 50 pg, 2.5 pg to 50 pg, 5 pg to 50 pg, 10 pg to 50 pg, 25 pg to 50 pg, 0.5 pg to 25 pg, 1 pg to 25 pg, 2.5 pg to 25 pg, 5 pg to 25 pg, 10 pg to 25 pg, 0.5 pg to 10 pg, 1 pg to 10 pg, 2.5 pg to 10 pg, 5 pg to 10 pg, 0.5 pg to 5 ng, 1 pg to 5 ng, 2.5 pg to5 pg, 0.5 pg to 2.5 pg, 1 pg to 2.5 pg, or 0.5 p g to 1 pg. Other amounts are contemplated.

In certain embodiments, the method comprises an amount of genomic DNA to be amplified of 1 pg to 6 ng.

In certain embodiments, the genomic DNA comprises DNA from 1 to 50 cells. In certain embodiments, the genomic DNA comprises DNA from a single cell, 1 to 10 cells, 1 to 20 cells, 1 to 50 cells, more than 10 cells, less than 10 cells, more than 50 cells, less than 50 cells, more than 100 cells, or less than 100 cells. DNA from other cell numbers is contemplated.

Examples of types of cells include one or more embryonic cells, an oocyte or a polar body thereof, sperm, one or more germ cells, one or more somatic cells, one or more human or animal cells, one or more plant cells, one or more cells from a microorganism, one or more cells for screening for a disease, condition or state, one or more cancerous or pre-cancerous cells, cells from a biopsy, one or more fetal cells, cells from a tissue sample, cells in/from a bodily fluid, cells in/from a blood sample, cells in/from amniotic fluid, cells in/from a formalin fixed sample, cells in/from a paraffin embedded sample, and cells in/from a swab. Other types of cells, and sources of cells, are contemplated. Methods for obtaining cells are known in the art.

The term "degenerate oligonucleotide primer" refers to an oligonucleotide with all or part of its nucleotide sequence being a partially degenerate sequence. Such primers can potentially bind to many sequences. Typically, such oligonucleotides either have a part of their nucleotide sequence which has the possibility of more than one base at a particular position, and/or the oligonucleotide has a part of its nucleotide sequence with bases that can form base pairs with one or more other bases. Methods and programs for designing primers, including degenerate oligonucleotide primers, are known in the art.

In certain embodiments, the amplification of genomic DNA using degenerate oligonucleotide primers results in whole genome amplification (WGA) of the genomic DNA.

The degenerate oligonucleotide primer may include DNA, RNA, a variant or DNA or RNA, a variant of the sugar-phosphate backbone, and/or a variant of one or more bases, such as a modified base, or a methylated base. Other types of variants are contemplated.

Examples of degenerate bases with the ability to bind to more than one base include 2-Amino Purine, 5-methyl isodeoxycytosine (Me isodC), 5-nitroindole, Inosine deoxy, Inosine ribo, iso deoxyguanosine dG. Other bases are contemplated.

Methods for designing degenerate primers are known in the art, and include programs such as iCODEHOP (COnsensus-DEgenerate Hybrid Oligonucleotide Primers), NCBI Primer-BLAST and HYDEN (HighlY DEgeNerate primers). Methods for producing degenerate oligonucleotide primers are known in the art, including chemical synthesis.

In certain embodiments, the one or more degenerate oligonucleotide primers comprise a sequence of one or more defined/fixed nucleotides at its 3' end. In certain embodiments, the degenerate oligonucleotide primer comprises a sequence of one or more defined/fixed nucleotides at its 3'end and at its 5'end.

In certain embodiments, the one or more degenerate oligonucleotide primers comprise a nucleotide sequence comprising 6 to 12 contiguous random nucleotides. For example, a degenerate oligonucleotide primer for use in WGA is a primer with the sequence 5'-CTCGAGNNNNATGTGG-3' (SEQ ID NO. 5) (wherein N=A, C, G or T), a primer with the sequence 5'-CGACTCGAGNNNNNNATGTGG-3' (SEQ ID NO. 6) (wherein N=A, C, G or T), or a primer with the sequence 5'-CCGACCGAGNNNNNNATGTGG-3' (SEQ ID NO. 7) (wherein N=A, C, G or T). Primers with other sequences are contemplated.

In certain embodiments, the degenerate oligonucleotide primer has a size of at least 12 bases, at least 13 bases, at least 14 bases, at least 15 bases, at least 16 bases, at least 17 bases, at least 18 bases, at least 19 bases, at least 20 bases, or at least 25 bases. Other sizes are contemplated.

In certain embodiments, the degenerate oligonucleotide primer has a size less than 25 bases, less than 20 bases, less 19 bases, less than 18 bases, less than 17 bases, less than 16 bases, less than 15 bases, less than 14 bases, less than 13 bases or less than 12 bases. Other sizes are contemplated.

In certain embodiments, the degenerate oligonucleotide primer has a size from 12 to 25 bases, 13 to 25 bases, 14 to 25 bases, 15 to 25 bases, 16 to 25 bases, 17 to 25 bases, 18 to 25 bases, 19 to 25 bases, 20 to 25 bases, 21 to 25 bases, 22 to 25 bases, 23 to 25 bases, 24 to 25 bases, 12 to 24 bases, 13 to 24 bases, 14 to 24 bases, 15 to 24 bases, 16 to 24 bases, 17 to 24 bases, 18 to 24 bases, 19 to 24 bases, 20 to 24 bases, 21 to 24 bases, 22 to 24 bases, 23 to 24 bases, 12 to 23 bases, 13 to 23 bases, 14 to 23 bases, 15 to 23 bases, 16 to 23 bases, 17 to 23 bases, 18 to 23 bases, 19 to 23 bases, 20 to 23 bases, 21 to 23 bases, 22 to 23 bases, 12 to 22 bases, 13 to 22 bases, 14 to 22 bases, 15 to 22 bases, 16 to 22 bases, 17 to 22 bases, 18 to 22 bases, 19 to 22 bases, 20 to 22 bases, 21 to 22 bases, 12 to 20 bases, 13 to 20 bases, 14 to 20 bases, 15 to 20 bases, 16 to 20 bases, 17 to 20 bases, 18 to 20 bases, 19 to 20 bases, 12 to 19 bases, 13 to 19 bases, 14 to 19 bases, 15 to 19 bases, 16 to 19 bases, 17 to 19 bases, 18 to 19 bases, 12 to 18 bases, 13 to 18 bases, 14 to 18 bases, 15 to 18 bases, 16 to 18 bases, 17 to 18 bases, 12 to 17 bases, 13 to 17 bases, 14 to 17 bases, 15 to 17 bases, 16 to 17 bases, 12 to 16 bases, 13 to 16 bases, 14 to 16 bases, 15 to 16 bases, 12 to 15 bases, 13 to 15 bases, 14 to 15 bases, 12 to 14 bases, 13 to 14 bases o 12 to 13 bases. Other sizes are contemplated.

Methods for using degenerate oligonucleotide primers are known in the art, for example as described in Telenius, H., Carter, N.P., Bebb, C.E., *et al.* (1992) "Degenerate oligonucleotide-primed PCR: general amplification of target DNA by a single degenerate primer" *Genomics* 13: 718.

In certain embodiments, the one or more degenerate oligonucleotide primers comprise a set of primers with the same general nucleotide sequence. In certain embodiments, the one or more degenerate oligonucleotide primers comprise a nucleotide sequence of the same general formula. In certain embodiments, the one or more degenerate primers comprise a set of primers with a different general nucleotide sequence. In certain embodiments, the one or more degenerate oligonucleotide primers are different.

In certain embodiments, the method comprises a concentration of the one or more degenerate oligonucleotide primers of at least 0.1 µM, at least 0.25 µM, at least 0.5 µM, at least 1 µM, at least 2.5 µM, or at least 5 µM. Other concentrations are contemplated.

In certain embodiments, the method comprises a concentration of the one or more degenerate oligonucleotide primers of less than 0.1 µM, less than 0.25 µM, less than 0.5 µM, less than 1 µM, less than 2.5 µM, or less than 5 µM. Other concentrations are contemplated.

In certain embodiments, the method comprises a concentration of the one or more degenerate oligonucleotide primers of 0.05 to 5 µM, 0.1 to 5 µM, 0.25 to 5 µM, 0.5 to 5 µM, 1 to 5 µM, 2.5 to 5 µM, 0.05 to 4 µM, 0.1 to 4 µM, 0.25 to 4 µM, 0.5 to 4 µM, 1 to 4 µM, 2.5 to 4 µM, 0.05 to 2.5 µM, 0.1 to 2.5 µM, 0.25 to 2.5 µM, 0.5 to 2.5 µM, 1 to 2.5 µM, 0.05 to 1 µM, 0.1 to 1 µM, 0.25 to 1 µM, 0.5 to 1 µM, 0.05 to 0.5 µM, 0.1 to 0.5 µM, 0.25 to 0.5 µM, 0.05 to 0.25 µM, 0.1 to 0.25 µM, or 0.05 to 0.1 µM. Other concentrations are contemplated.

In certain embodiments, the method comprises a concentration of the one or more degenerate oligonucleotide primers of 0.1 to 4 µM.

In certain embodiments, the one or more target DNA sequence specific primers have a size of at least 12 bases, at least 13 bases, at least 14 bases, at least 15 bases, at least 16 bases, at least 17 bases, at least 18 bases, at least 19 bases, at least 20 bases, or at least 25 bases. Other sizes are contemplated.

In certain embodiments, the one or more target DNA sequence specific primers have a size less than 25 bases, less than 20 bases, less 19 bases, less than 18 bases, less than 17 bases, less than 16 bases, less than 15 bases, less than 14 bases, less than 13 bases or less than 12 bases. Other sizes are contemplated.

In certain embodiments, the one or more target DNA sequence specific primers have a size from 12 to 25 bases, 13 to 25 bases, 14 to 25 bases, 15 to 25 bases, 16 to 25 bases, 17 to 25 bases, 18 to 25 bases, 19 to 25 bases, 20 to 25 bases, 21 to 25 bases, 22 to 25 bases, 23 to 25 bases, 24 to 25 bases, 12 to 24 bases, 13 to 24 bases, 14 to 24 bases, 15 to 24 bases, 16 to 24 bases, 17 to 24 bases, 18 to 24 bases, 19 to 24 bases, 20 to 24 bases, 21 to 24 bases, 22 to 24 bases, 23 to 24 bases, 12 to 23 bases, 13 to 23 bases, 14 to 23 bases, 15 to 23 bases, 16 to 23 bases, 17 to 23 bases, 18 to 23 bases, 19 to 23 bases, 20 to 23 bases, 21 to 23 bases, 22 to 23 bases, 12 to 22 bases, 13 to 22 bases, 14 to 22 bases, 15 to 22 bases, 16 to 22 bases, 17 to 22 bases, 18 to 22 bases, 19 to 22 bases, 20 to 22 bases, 21 to 22 bases, 12 to 20 bases, 13 to 20 bases, 14 to 20 bases, 15 to 20 bases, 16 to 20 bases, 17 to 20 bases, 18 to 20 bases, 19 to 20 bases, 12 to 19 bases, 13 to 19 bases, 14 to 19 bases, 15 to 19 bases, 16 to 19 bases, 17 to 19 bases, 18 to 19 bases, 12 to 18 bases, 13 to 18 bases, 14 to 18 bases, 15 to 18 bases, 16 to 18 bases, 17 to 18 bases, 12 to 17 bases, 13 to 17 bases, 14 to 17 bases, 15 to 17 bases, 16 to 17 bases, 12 to 16 bases, 13 to 16 bases, 14 to 16 bases, 15 to 16 bases, 12 to 15 bases, 13 to 15 bases, 14 to 15 bases, 12 to 14 bases, 13 to 14 bases o 12 to 13 bases. Other sizes are contemplated.

Methods for designing and producing primers are known in the art. Typically, primers/oligonucleotides are produced using chemical synthesis from nucleoside phosphoramidites, but other methods of chemical synthesis are contemplated.

In certain embodiments, the one or more target DNA sequence specific primers comprise a melting temperature of at least 50°C, at least 55°C, at least 60°C or at least 65°C. Other melting temperatures are contemplated.

Methods for determining melting temperatures are known in the art. The melting temperatures quoted herein are provided as a certain temperature (Tₘ) under the amplification conditions being used. It will be appreciated that the Tm value is dependent upon a number of factors, including salt concentration and Mg²⁺ concentration, and a variety of different types of calculations are known in the art to determine Tₘ.

In certain embodiments, the one or more target DNA sequence specific primers comprise a melting temperature of less than 50°C, less than 55°C, less than 60°C or less than 65°C. Other melting temperatures are contemplated.

In certain embodiments, the one or more target DNA sequence specific primers comprise a melting temperature in the range from 45°C to 65°C, 50°C to 65°C, 55oC to 65°C, 60°C to 65°C, 45°C to 60°C, 50°C to 60°C, 55°C to 60°C, 45°C to 55°C, 50°C to 55°C or 45°C to 50°C. Other melting temperatures are contemplated.

In certain embodiments, the one or more target DNA sequence specific primers comprise a GC content of at least 18%, at least 20%, at least 25%, at least 30%, at least 40%, at least 45%, at least 50%, at least 55%, at least 60%, or at least 65%. Other levels of GC content are contemplated. Methods for determining GC content are known in the art.

In certain embodiments, the one or more target DNA sequence specific primers comprise a GC content of less than 18%, less than 20%, less than 25%, less than 30%, less than 40%, less than 45%, less than 50%, less than 55%, less than 60%, or less than 65%. Other levels of GC content are contemplated.

In certain embodiments, the one or more target DNA sequence specific primers comprise a GC content of 18% to 65%, 20% to 65%, 25% to 65%, 25% to 65%, 30% to 65%, 35% to 65%, 40% to 65%, 45% to 65%, 50% to 65%, 55% to 65%, 60% to 65%, 18% to 60%, 20% to 60%, 25% to 60%, 25% to 60%, 30% to 60%, 35% to 60%, 40% to 60%, 45% to 60%, 50% to 60%, 55% to 60%, 18% to 55%, 20% to 55%, 25% to 55%, 25% to 55%, 30% to 55%, 35% to 55%, 40% to 55%, 45% to 55%, 50% to 55%, 18% to 50%, 20% to 50%, 25% to 50%, 30% to 50%, 35% to 50%, 40% to 50%, 45% to 50%, 18% to 45%, 20% to 45%, 25% to 45%, 30% to 45%, 35% to 45%, 40% to 45%, 18% to 40%, 20% to 40%, 25% to 40%, 30% to 40%, 35% to 40%, 18% to 35%, 20% to 35%, 25% to 35%, 30% to 35%, 18% to 30%, 20% to 30%, 25% to 30%, 18% to 25%, 20% to 25%, or 18% to 20%. Other levels of GC content are contemplated.

In certain embodiments, the one or more target DNA sequence specific primers comprise a melting temperature of 50° to 64°C and a GC content of 18% to 65%.

In certain embodiments, the method comprises a concentration of the one or more target DNA sequence specific primers of at least 0.05 µM, at least 0.1 µM, at least 0.25 µM, at least 0.5 µM, at least 1 µM, at least 2.5 µM, or at least 5 µM. Other concentrations are contemplated.

In certain embodiments, the method comprises a concentration of the one or more target DNA sequence specific primers of less than 0.05 µM, less than 0.1 µM, less than 0.25 µM, less than 0.5 µM, less than 1 µM, less than 2.5 µM, or less than 5 µM. Other concentrations are contemplated.

In certain embodiments, the method comprises a concentration of the one or more target DNA sequence specific primers of 0.05 to 5 µM, 0.1 to 5 µM, 0.25 to 5 µM, 0.5 to 5 µM, 1 to 5 µM, 2.5 to 5 µM, 0.05 to 4 µM, 0.1 to 4 µM, 0.25 to 4 µM, 0.5 to 4 µM, 1 to 4 µM, 2.5 to 4 µM, 0.05 to 2.5 µM, 0.1 to 2.5 µM, 0.25 to 2.5 µM, 0.5 to 2.5 µM, 1 to 2.5 µM, 0.05 to 1 µM, 0.1 to 1 µM, 0.25 to 1 µM, 0.5 to 1 µM, 0.05 to 0.5 µM, 0.1 to 0.5 µM, 0.25 to 0.5 µM, 0.05 to 0.25 µM, 0.1 to 0.25 µM, or 0.05 to 0.1 µM. Other concentrations are contemplated.

In certain embodiments, the method comprises a concentration of the one or more target DNA sequence specific primers of 0.05 to 4 µM.

In certain embodiments, genomic DNA is provided for amplification. In certain embodiments, genomic DNA is provided by way of a cell containing the DNA for amplification. In certain embodiments, genomic DNA is released from a cell for amplification.

In certain embodiments, the genomic DNA is substantially un-purified DNA. In certain embodiments, the genomic DNA is lysed cellular genomic DNA. In certain embodiments, the genomic DNA is from a lysed cell. In certain embodiments, the genomic DNA is substantially un-purified DNA. In certain embodiments, the genomic DNA is partially or substantially purified.

In certain embodiments, the method comprises a lysis step of a cell. In certain embodiments, the method comprises a lysis step of a cell prior to amplification. Methods for lysis of cells are known in the art.

In certain embodiments, the method does not comprise a separate lysis step. In certain embodiments, the method comprises lysis of a cell and amplification of genomic DNA concurrently. In certain embodiments, amplification of genomic DNA from a cell occurs without a separate lysis step. In certain embodiments, genomic DNA from a cell is made available for amplification without a separate lysis step.

In certain embodiments, genomic DNA from a cell is made available for amplification as part of the amplification procedure.

In certain embodiments, the method comprises amplifying the genomic DNA with the one or more degenerate oligonucleotide primers for at least one or more cycles of the amplification with the target DNA specific primers.

In certain embodiments, the method comprises amplifying with the one or more target DNA sequence specific primers for the duration of the amplifying of the genomic DNA with the one or more degenerate oligonucleotide primers.

In certain embodiments, the method comprises addition of the one or more degenerate oligonucleotide primers and the target DNA sequence specific primers prior to amplification of the genomic DNA.

In certain embodiments, the method comprises addition of the one or more degenerate oligonucleotide primers and the target DNA sequence specific primers at the same time.

In certain embodiments, the method comprises addition of the one or more degenerate oligonucleotide primers and subsequently the addition of the target DNA sequence specific primers.

In certain embodiments, the method comprises amplifying the genomic DNA with the one or more degenerate oligonucleotide primers and subsequently amplifying the genomic DNA with the one or more degenerate oligonucleotide primers and the one or more target DNA sequence specific primers.

In certain embodiments, the method comprises addition of the one or more target DNA sequence specific primers to genomic DNA amplified with the one or more degenerate oligonucleotide primers and subsequently amplifying with the one or more target DNA sequence specific primers and the one or more degenerate oligonucleotide primers.

In certain embodiments, the method comprises a polymerase chain reaction comprising one or more cycles of a low stringency amplification and one or more further cycles of higher stringency amplification.

In certain embodiments, the method comprises amplifying with the one or more degenerate oligonucleotide primers for one or more cycles of a low stringency amplification.

In certain embodiments, the method comprises amplifying with the one or more degenerate oligonucleotide primers and the target DNA sequences specific primers for one or more cycles of a higher stringency amplification.

In certain embodiments, the method comprises amplifying with the one or more degenerate oligonucleotide primers for the one or more cycles of low stringency amplification and amplifying with the one or more degenerate oligonucleotide primers and the target DNA sequences specific primers for the one or more cycles of higher stringency amplification.

In certain embodiments, the amplifying comprises a polymerase chain reaction comprising one or more cycles with an annealing temperature below 35°C.

In certain embodiments, the method comprises amplifying with the one or more degenerate oligonucleotide primers for the one or more cycles with an annealing temperature below 35°C.

In certain embodiments, the amplifying comprises a polymerase chain reaction comprising 1 to 8 cycles with an annealing temperature below 35°C.

In certain embodiments, the method comprises amplifying with the one or more degenerate oligonucleotide primers for the 1 to 8 cycles with an annealing temperature below 35°C.

Other temperatures and cycling conditions are contemplated.

In certain embodiments, the amplifying comprises a polymerase chain reaction comprising one or more cycles with an annealing temperature of 20° to 35°C.

In certain embodiments, the method comprises amplifying with the one or more degenerate oligonucleotide primers for one or more cycles with an annealing temperature of 20°C to 35°C.

In certain embodiments, the amplifying comprises a polymerase chain reaction comprising 1 to 8 cycles with an annealing temperature of 20° to 35°C. Other temperatures and cycling conditions are contemplated.

In certain embodiments, the method comprises amplifying with the one or more degenerate oligonucleotide primers for 1 to 8 cycles with an annealing temperature of 20°C to 35°C.

In certain embodiments, the amplifying comprises a polymerase chain reaction comprising one or more cycles with an annealing temperature above 48°C or 50°C.

In certain embodiments, the method comprises amplifying with the one or more degenerate oligonucleotide primers and the one or more target DNA sequence specific primers for one or more cycles with an annealing temperature above 48°C or 50°C.

In certain embodiments, the amplifying comprises a polymerase chain reaction comprising 5 to 25 cycles with an annealing above 48°C or 50°C. Other temperatures and cycling conditions are contemplated.

In certain embodiments, the method comprises amplifying with the one or more degenerate oligonucleotide primers and the one or more target DNA sequence specific primers for 5 to 25 cycles with an annealing temperature above 48°C or 50°C.

In certain embodiments, the amplifying comprises a polymerase chain reaction comprising one or more cycles with an annealing temperature of one of 48° to 68°C, 52° to 65°C or 52° to 62°C.

In certain embodiments, the method comprises amplifying with the one or more degenerate oligonucleotide primers and the one or more target DNA sequence specific primers for one or more cycles with an annealing temperature of one of 48° to 68°C, 52° to 65°C or 52° to 62°C.

In certain embodiments, the amplifying comprises a polymerase chain reaction comprising 5 to 25 cycles with an annealing temperature of one of 48° to 68°C, 52° to 65°C or 52° to 62°C. Other temperatures and cycling conditions are contemplated.

In certain embodiments, the method comprises amplifying with the one or more degenerate oligonucleotide primers and the one or more target DNA sequence specific primers for 5 to 25 cycles with an annealing temperature of one of 48° to 68°C, 52° to 65°C or 52° to 62°C.

In certain embodiments, the amplifying comprises a polymerase chain reaction comprising one or more cycles with an annealing temperature below 35°C and one or more further cycles with an annealing temperature above 50°C. Other temperatures and cycling conditions are contemplated.

In certain embodiments, the amplifying comprises a polymerase chain reaction comprising one or more cycles with an annealing temperature below 35°C with the one or more degenerate oligonucleotide primers and one or more further cycles with an annealing temperature above 48°C or 50°C with the one or more degenerate oligonucleotide primers and the one or more target DNS specific primers. Other temperatures and cycling conditions are contemplated.

In certain embodiments, the amplifying comprises a polymerase chain reaction comprising one or more cycles with an annealing temperature of 20° to 35°C and one or more further cycles with an annealing temperature of one of 48° to 68°C, 52° to 65°C or 52° to 62°C. Other temperatures are contemplated.

In certain embodiments, the amplifying comprises a polymerase chain reaction comprising one or more cycles with an annealing temperature of 20° to 35°C with the one or more degenerate oligonucleotide primers and one or more further cycles with an annealing temperature of one of 48° to 68°C, 52° to 65°C or 52° to 62°C with the one or more degenerate oligonucleotide primers and the one or more target DNA sequence specific primers. Other temperatures are contemplated.

In certain embodiments, the amplifying comprises a polymerase chain reaction comprising 1 to 8 cycles with an annealing temperature of 20° to 35°C and 5 to 25 cycles with an annealing temperature of one of 48° to 68°C, 52° to 65°C or 52° to 62°C for 5 to 25 cycles. Other temperatures and cycling conditions are contemplated.

In certain embodiments, the amplifying comprises a polymerase chain reaction comprising 1 to 8 cycles with an annealing temperature of 20° to 35°C with the degenerate oligonucleotide primers and 5 to 25 cycles with an annealing temperature of one of 48° to 68°C, 52° to 65°C or 52° to 62°C for 5 to 25 cycles with the one or more degenerate oligonucleotide primers and the one or more target DNA sequence specific primers.

Examples of polymerases are described herein.

In certain embodiments, the method comprises use of one or more of a Taq polymerase (available from, for example, New England Biolabs), a Phi29 polymerase (available from, for example, New England Biolabs), Pfu DNA polymerase (available from, for example, Promega), a Bst DNA polymerase (available from, for example, New England Biolabs), VentR^{™} and Deep VentR^{™} DNA polymerases (available from, for example, New England Biolabs), 9°Nm DNA polymerase (available from, for example, New England Biolabs), Herculase II Fusion DNA Polymerases (available from Agilent Technologies), KAPA HiFi (available from Kapa Biosystems), KAPA2G Robust (available from Kapa Biosystems), Klenow fragment of DNA polymerase I (available from, for example, New England Biolabs), PhiPRDl DNA polymerase (Jung et al. (1987), Proc. Natl. Acad. Sci. USA 84:8287), phage M2 DNA polymerase (Matsumoto et al. (1989) Gene 84:247), T4 DNA polymerase (available from, for example, New England Biolabs) and T5 DNA polymerase (Chatterjee et al., (1991) Gene 97:13-19) . Other types of polymerases are contemplated and are commercially available. Suitable reaction conditions may be selected for a specific polymerase.

In certain embodiments, the method comprises use of polymerase with a processivity of 1 to 50 nucleotides per second at 72°C. Polymerases with a requisite processivity are known in the art.

In certain embodiments, the method comprises use of a polymerase with a fidelity of at least 285 x 10⁻⁶ errors per template nucleotide. In certain embodiments, the method comprises use of a polymerase with a fidelity of greater than 285 x 10⁻⁶ errors per template nucleotide. Polymerases with a requisite fidelity are known in the art.

In certain embodiments, the method comprises one or more further rounds of amplifying the amplified target DNA sequence.

In certain embodiments, the one or more further rounds of amplifying comprise 10 to 40 cycles. In certain embodiments, the one or more further rounds of amplifying comprise cycles with an annealing temperature of one of 48° to 68°C, 52° to 65°C or 52° to 62°C. In certain embodiments, the one or more further rounds of amplifying comprise 10 to 40 cycles with an annealing temperature of one of 48° to 68°C, 52° to 65°C or 52° to 62°C.

In certain embodiments, the method comprises one or more further rounds of amplifying the amplified target DNA sequence with one or more further target DNA sequence specific primers.

In certain embodiments, the further target DNA sequence specific primers are the same as the one or more target DNA sequence primers.

In certain embodiments, the further target DNA sequence specific primers are different to the one or more target DNA sequence primers.

In certain embodiments, the further target DNA sequence primers are nested primers.

Methods for designing and producing the further primers, including nested primers, are known in the art.

In certain embodiments, the method comprising amplifying two or more target DNA sequences.

In certain embodiments, the method comprises amplifying two or more target DNA sequences from the genomic DNA, by amplifying the genomic DNA with one or more degenerate oligonucleotide primers in the presence of primers specific for the two or more target DNA sequences.

In certain embodiments, the method comprises amplifying a target DNA sequence from genomic DNA by amplifying the genomic DNA with one or more degenerate oligonucleotide primers in the presence of one or more target DNA sequence specific primers, and subsequently amplifying one or more other target DNA sequences.

In certain embodiments, the method further comprises assessing nucleotide content of the amplified target DNA sequence. Methods for assessing nucleotide content are known in the art.

In certain embodiments, the assessing of the nucleotide content comprises detecting the presence or absence of a polymorphism, a mutation, a deletion, an insertion, a translocation, methylation status, and/or an epigenetic change. Other methods for assessing nucleotide content are contemplated.

In certain embodiments, the assessing of the nucleotide content comprises sequencing all or part of the amplified target DNA sequence.

In certain embodiments, the method further comprises assessing nucleotide content by one or more of sequencing all or part of the amplified target DNA sequence, hybridization of a strand of the amplified target DNA sequence to one or more oligonucleotides, real time PCR, and high resolution melt analysis. Other methods are contemplated.

In certain embodiments, the method may be used for genetic screening, preimplantation genetic screening, preimplantation genetic diagnosis, prenatal screening, screening for a disease, screening for a cancer, screening for genetic markers, and/or forensic screening. Other uses are contemplated.

Certain embodiments of the present disclosure provide a method of amplifying a target DNA sequence from genomic DNA as described herein, by amplifying the target DNA sequence with one or more target DNA sequence specific primers for at least one or more cycles of amplification of genomic DNA with one or more degenerate oligonucleotide primers.

Certain embodiments of the present disclosure provide a method of amplifying a target DNA sequence from genomic DNA, the method comprising amplifying the target DNA sequence with one or more target DNA sequence specific primers for at least one or more cycles of amplification of genomic DNA with one or more degenerate oligonucleotide primers, thereby amplifying the target DNA sequence.

Certain embodiments of the present disclosure provide a method of amplifying a target DNA sequence from genomic DNA, the method comprising:
amplifying genomic DNA with one or more degenerate oligonucleotide primers; and
for at least one or more cycles of the amplification of the genomic DNA also amplifying the target DNA sequence with one or more target DNA sequence specific primers,
thereby amplifying the target DNA sequence.

Methods of amplification are as described herein. In certain embodiments, the amplifying comprises a polymerase chain reaction, as described herein.

Certain embodiments of the present disclosure provide a method of amplifying a target DNA sequence from genomic DNA using whole genome amplification, as described herein.

Certain embodiments of the present disclosure a method of amplifying a target DNA sequence from genomic DNA, by performing whole genome amplification of the genomic DNA with one or more degenerate oligonucleotide primers; and for at least part of the whole genome amplification also amplifying the target DNA sequence with one or more target DNA sequence specific primers.

Certain embodiments of the present disclosure provide a method of amplifying a target DNA sequence from genomic DNA, the method comprising:
performing whole genome amplification of the genomic DNA with one or more degenerate oligonucleotide primers; and
for at least part of the whole genome amplification also amplifying the target DNA sequence with one or more target DNA sequence specific primers,
thereby amplifying the target DNA sequence.

In certain embodiments, the whole genome amplification comprises amplifying using a polymerase chain reaction.

Methods for whole genome amplification are as described herein. For example, whole genome application may be performed as described in Arneson N., Hughes S., Houlston R. and Done S. (2007) "PCR-Based Whole Genome Amplification," Chapter 18, PCR (eds. Hughes and Moody). Scion Publishing Ltd., Oxfordshire, UK, 2007.

Certain embodiments of the present disclosure provide a target DNA sequence amplified by a method as described herein.

In certain embodiments, a target DNA sequence may be further amplified. In certain embodiments, the target DNA sequence may be further amplified with the same primers. In certain embodiments, the target DNA sequence may be further amplified with one or more other target DNA sequence specific primers. Methods for amplification with one or more other/further target DNA sequence specific primers are as described herein.

Certain embodiments provide use of a target DNA sequence amplified as described herein.

Certain embodiments of the present disclosure provide use of a target DNA sequence for genetic screening, genetic diagnosis, preimplantation genetic screening, preimplantation genetic diagnosis, prenatal screening, screening for a disease, screening for a cancer, screening for a genetic marker, screening for a polymorphism, a mutation, a deletion, an insertion, a translocation, an expansion, an inversion, methylation status, and/or an epigenetic change, and/or forensic screening. Other uses are contemplated.

Certain embodiments of the present disclosure provide a method of assessing nucleotide content of a target DNA sequence amplified from genomic DNA by a method as described herein. Methods for assessing nucleotide content are as described herein.

Certain embodiments of the present disclosure provide a method of assessing nucleotide content of a target DNA sequence by assessing the nucleotide content of a target DNA sequence amplified by a method as described herein.

Certain embodiments of the present disclosure provide a method of assessing nucleotide content of a target DNA sequence in genomic DNA, the method comprising:
amplifying the genomic DNA with one or more degenerate oligonucleotide primers in the presence of one or more target DNA sequence specific primers to produce an amplified target DNA sequence; and
assessing the nucleotide content of the amplified target DNA sequence so as to assess the nucleotide content of the target DNA sequence.

Certain embodiments of the present disclosure provide a method of assessing nucleotide content of a target DNA sequence in genomic DNA, the method comprising amplifying the target DNA sequence with one or more target DNA sequence specific primers for at least one or more cycles of amplification of genomic DNA with one or more degenerate oligonucleotide primer to produce an amplified target DNA sequence, and assessing the nucleotide content of the amplified target DNA sequence so as to assess the nucleotide content of the target DNA sequence.

Certain embodiments of the present disclosure provide a method of assessing nucleotide content of a target DNA sequence in genomic DNA, the method comprising:
amplifying genomic DNA with one or more degenerate oligonucleotide primers; and
for at least one or more cycles of the amplification of the genomic DNA also amplifying the target DNA sequence with one or more target DNA sequence specific primers to produce an amplified target DNA sequence; and
assessing the nucleotide content of the amplified target DNA sequence so as to assess the nucleotide content of the target DNA sequence.

Certain embodiments of the present disclosure provide a method of assessing nucleotide content of a target DNA sequence in genomic DNA, the method comprising:
performing whole genome amplification of the genomic DNA with one or more degenerate oligonucleotide primers;
for at least part of the whole genome amplification also amplifying the target DNA sequence with one or more target DNA sequence specific primers to produce an amplified target DNA sequence; and
assessing the nucleotide content of the amplified target DNA sequence so as to assess the nucleotide content of the target DNA sequence.

Methods for assessing the nucleotide content are as described herein. In certain embodiments, the assessing of the nucleotide content comprises determining the nucleotide sequence of all or part of the amplified target DNA.

In certain embodiments, the methods for assessing the nucleotide content comprise detecting the presence or absence of a polymorphism, a mutation, a deletion, an insertion, a translocation, methylation status, and/or an epigenetic change.

In certain embodiments, the methods for assessing the nucleotide content comprise one or more of sequencing all or part of the amplified target DNA sequence, hybridization of a strand of the amplified target DNA sequence to one or more oligonucleotides, real time PCR, and high resolution melt analysis. Other methods are contemplated.

In certain embodiments, the methods for assessing the nucleotide content are used for genetic screening, genetic diagnosis, preimplantation genetic screening, preimplantation genetic diagnosis, prenatal screening, screening for a disease, screening for a cancer, screening for genetic markers, and/or forensic screening. Other uses are contemplated.

Certain embodiments of the present disclosure provide a method of determining the sequence of all or part of a target DNA sequence in genomic DNA, by amplifying the target DNA sequence as described herein and determining the nucleotide sequence of all or part of the amplified target DNA sequence.

Certain embodiments of the present disclosure provide a method of determining the nucleotide sequence of a target DNA sequence in genomic DNA, the method comprising:
amplifying the genomic DNA with one or more degenerate oligonucleotide primers in the presence of one or more target DNA sequence specific primers to produce an amplified target DNA sequence; and
determining the nucleotide sequence of the amplified target DNA sequence,
thereby determining the nucleotide sequence of the target DNA sequence.

In certain embodiments, the method comprises determining the nucleotide sequence of substantially all the nucleotides in the amplified target DNA sequence. In certain embodiments, the method comprises determining the nucleotide sequence of one or more nucleotides in the amplified target DNA sequence.

Methods for determining the nucleotide sequence of a DNA are known in the art. Examples include techniques based on dideoxy chain termination, Next-Generation Sequencing methods, shot gun sequencing, hybridization to one or more oligonucleotides, real time PCR, and high resolution melt analysis. Other methods are contemplated.

In certain embodiments, the methods as described herein are used for genetic screening or diagnosis.

Certain embodiments of the present disclosure provide a method of genetic screening or diagnosis, by amplifying a target DNA sequence as described herein and assessing the amplified target DNA sequence for screening or diagnosis.

Certain embodiments of the present disclosure provide a method of genetic screening or diagnosis of an embryo. Genetic screening or diagnosis of other cells is contemplated and is described herein.

Certain embodiments of the present disclosure provide a method of genetic screening of an embryo, the method comprising:
amplifying the genomic DNA from the embryo with one or more degenerate oligonucleotide primers in the presence of one or more target DNA sequence specific primers to produce an amplified target DNA sequence; and
assessing the amplified target DNA sequence to screen the embryo.

Methods for obtaining one or more cells from an embryo for analysis are known in the art. Methods for amplifying the genomic DNA from an embryo with one or more degenerate oligonucleotide primers in the presence of one or more target DNA sequence specific primers to produce an amplified target DNA sequence are as described herein.

In certain embodiments, the assessing of the amplified target DNA sequence comprises determining the nucleotide content of the amplified target DNA sequence.

In certain embodiments, the assessing of the amplified target DNA sequence comprises detecting the presence or absence of a polymorphism, a mutation, a deletion, an insertion, a translocation, methylation status, and/or an epigenetic change.

In certain embodiments, the assessing comprises one or more of sequencing all or part of the amplified target DNA sequence, hybridization of a strand of the amplified target DNA sequence to one or more oligonucleotides, real time PCR, and high resolution melt analysis. Other methods are contemplated.

In certain embodiments, the methods as described herein are used for screening of an embryo, such as pre-implantation genetic screening.

Certain embodiments of the present disclosure provide a method of pre-implantation genetic screening of an embryo, for example for suitability of implantation, for the presence or absence of a genetic disease, and/or for the presence or absence of desired and/or undesired mutations. Other types of screening are contemplated.

Certain embodiments of the present disclosure provide a method of pre-implantation genetic screening of an embryo, the method comprising:
amplifying the genomic DNA from the embryo with one or more degenerate oligonucleotide primers in the presence of one or more target DNA sequence specific primers to produce an amplified target DNA sequence; and
assessing the amplified target DNA sequence to screen the embryo.

Certain embodiments of the present disclosure provide a method of pre-implantation genetic screening of an embryo, the method comprising amplifying a target DNA sequence from the embryo with one or more target DNA sequence specific primers for at least one or more cycles of amplification of genomic DNA from the embryo with one or more degenerate oligonucleotide primers to produce an amplified target DNA sequence, and assessing the amplified target DNA sequence to screen the embryo.

Certain embodiments of the present disclosure provide a method of pre-implantation genetic screening of an embryo, the method comprising:
amplifying genomic DNA from the embryo with one or more degenerate oligonucleotide primers;
for at least one or more cycles of the amplification of the genomic DNA also amplifying a target DNA sequence from the embryo with one or more target DNA sequence specific primers to produce an amplified target DNA sequence; and
assessing the amplified target DNA sequence to screen the embryo.

Certain embodiments of the present disclosure provide a method of pre-implantation genetic screening of an embryo, the method comprising:
performing whole genome amplification of the genomic DNA from the embryo with one or more degenerate oligonucleotide primers;
for at least part of the whole genome amplification also amplifying a target DNA sequence from the embryo with one or more target DNA sequence specific primers to produce an amplified target DNA sequence; and
assessing the amplified target DNA sequence to screen the embryo.

In certain embodiments, the methods as described herein are used for screening of an oocyte, such as genetic screening. For example, an oocyte may be screened for its suitability for fertilization, suitability for implantation, for the presence or absence of a genetic disease, for its mitochondrial content or genome, and/or for the presence or absence of desired and/or undesired mutations. Other types of screening are contemplated.

Certain embodiments of the present disclosure provide a method of genetic screening of an oocyte.

Methods for obtaining an oocyte or a polar body thereof for analysis are known in the art. Methods for amplifying the genomic DNA from an oocyte (or a polar body thereof) with one or more degenerate oligonucleotide primers in the presence of one or more target DNA sequence specific primers to produce an amplified target DNA sequence are as described herein.

In certain embodiments, the assessing of the amplified target DNA sequence comprises determining the nucleotide content of the amplified target DNA sequence.

In certain embodiments, the assessing of the amplified target DNA sequence comprises detecting the presence or absence of a polymorphism, a mutation, a deletion, an insertion, a translocation, methylation status, and/or an epigenetic change.

In certain embodiments, the assessing comprises one or more of sequencing all or part of the amplified target DNA sequence, hybridization of a strand of the amplified target DNA sequence to one or more oligonucleotides, real time PCR, and high resolution melt analysis. Other methods are contemplated.

In certain embodiments, the methods as described herein are used for pre-implantation genetic screening of an oocyte.

In certain embodiments, the method is used to screen an oocyte for suitability for fertilization.

Certain embodiments of the present disclosure provide a method pre-implantation genetic screening of an oocyte.

Certain embodiments of the present disclosure provide a method of pre-implantation genetic screening of an oocyte, the method comprising:
amplifying the genomic DNA from an oocyte or a polar body thereof with one or more degenerate oligonucleotide primers in the presence of one or more target DNA sequence specific primers to produce an amplified target DNA sequence; and
assessing the amplified target DNA sequence to screen the oocyte.

Certain embodiments of the present disclosure provide a method of pre-implantation genetic screening of an oocyte, the method comprising amplifying a target DNA sequence from the oocyte or a polar body thereof with one or more target DNA sequence specific primers for at least one or more cycles of amplification of genomic DNA from the oocyte or polar body with one or more degenerate oligonucleotide primers to produce an amplified target DNA sequence, and assessing the amplified target DNA sequence to screen the oocyte.

Certain embodiments of the present disclosure provide a method of pre-implantation genetic screening of an oocyte, the method comprising:
amplifying genomic DNA from the oocyte or a polar body thereof with one or more degenerate oligonucleotide primers;
for at least one or more cycles of the amplification of the genomic DNA also amplifying a target DNA sequence from the oocyte or polar body with one or more target DNA sequence specific primers to produce an amplified target DNA sequence; and
assessing the amplified target DNA sequence to screen the oocyte.

Certain embodiments of the present disclosure provide a method of pre-implantation genetic screening of an oocyte, the method comprising:
performing whole genome amplification of the genomic DNA from the oocyte or a polar body thereof with one or more degenerate oligonucleotide primers;
for at least part of the whole genome amplification also amplifying a target DNA sequence from the oocyte or polar body with one or more target DNA sequence specific primers to produce an amplified target DNA sequence; and
assessing the amplified target DNA sequence to screen the oocyte.

Methods for performing whole genome amplification are as described herein.

In certain embodiments, the methods as described herein are used for screening of sperm, such as genetic screening. For example, a sperm may be screened for its suitability for use in fertilization, for the presence or absence of a genetic disease, and/or for the presence or absence of desired and/or undesired mutations. Other types of screening are contemplated.

Certain embodiments of the present disclosure provide a method of genetic screening of sperm.

Certain embodiments of the present disclosure provide a method of genetic screening of sperm, the method comprising:
amplifying the genomic DNA from one or more sperm with one or more degenerate oligonucleotide primers in the presence of one or more target DNA sequence specific primers to produce an amplified target DNA sequence; and
assessing the amplified target DNA sequence to screen the sperm.

Methods for obtaining sperm for analysis are known in the art. Methods for amplifying the genomic DNA from sperm with one or more degenerate oligonucleotide primers in the presence of one or more target DNA sequence specific primers to produce an amplified target DNA sequence are as described herein.

In certain embodiments, the assessing of the amplified target DNA sequence comprises determining the nucleotide content of the amplified target DNA sequence.

In certain embodiments, the assessing of the amplified target DNA sequence comprises detecting the presence or absence of a polymorphism, a mutation, a deletion, an insertion, a translocation, methylation status, and/or an epigenetic change.

In certain embodiments, the assessing comprises one or more of sequencing all or part of the amplified target DNA sequence, hybridization of a strand of the amplified target DNA sequence to one or more oligonucleotides, real time PCR, and high resolution melt analysis.

Certain embodiments of the present disclosure provide a method of genetic screening of sperm, the method comprising amplifying a target DNA sequence from one or more sperm with one or more target DNA sequence specific primers for at least one or more cycles of amplification of genomic DNA from the one or more sperm with one or more degenerate oligonucleotide primers to produce an amplified target DNA sequence, and assessing the amplified target DNA sequence to screen the sperm.

Certain embodiments of the present disclosure provide a method of genetic screening of sperm, the method comprising:
amplifying genomic DNA from one or more sperm with one or more degenerate oligonucleotide primers;
for at least one or more cycles of the amplification of the genomic DNA also amplifying a target DNA sequence from the one or more sperm with one or more target DNA sequence specific primers to produce an amplified target DNA sequence; and
assessing the amplified target DNA sequence to screen the sperm.

Certain embodiments of the present disclosure provide a method of screening of sperm, the method comprising:
performing whole genome amplification of the genomic DNA from one or more sperm with one or more degenerate oligonucleotide primers;
for at least part of the whole genome amplification also amplifying a target DNA sequence from the one or more sperm with one or more target DNA sequence specific primers to produce an amplified target DNA sequence; and
assessing the amplified target DNA sequence to screen the sperm.

In certain embodiments, the methods as described herein are used for screening sperm for suitability for fertilization.

In certain embodiments, the methods as described herein are used for screening a fetus, such as genetic screening. For example, a fetus oocyte may be screened for the presence or absence of a genetic disease, for the presence or absence of genetic markers, for the presence or absence of chromosomal content, and/or for the presence or absence of desired and/or undesired mutations. Other types of screening are contemplated.

Certain embodiments of the present disclosure provide a method of screening of a fetus.

Certain embodiments of the present disclosure provide a method of prenatal screening of a fetus.

Certain embodiments of the present disclosure provide a method of prenatal screening of a fetus, the method comprising:
amplifying genomic DNA from the fetus with one or more degenerate oligonucleotide primers in the presence of one or more target DNA sequence specific primers to produce an amplified target DNA sequence; and
assessing the amplified target DNA sequence to screen the fetus.

Methods for obtaining genomic DNA from a fetus are known in art and include, sampling of amniotic fluid, chorionic villus sampling and obtaining cell-free DNA. Methods for amplifying the genomic DNA with one or more degenerate oligonucleotide primers in the presence of one or more target DNA sequence specific primers to produce an amplified target DNA sequence are as described herein.

In certain embodiments, the assessing of the amplified target DNA sequence comprises determining the nucleotide content of the amplified target DNA sequence.

In certain embodiments, the assessing of the amplified target DNA sequence comprises detecting the presence or absence of a polymorphism, a mutation, a deletion, an insertion, a translocation, methylation status, and/or an epigenetic change.

In certain embodiments, the assessing comprises one or more of sequencing all or part of the amplified target DNA sequence, hybridization of a strand of the amplified target DNA sequence to one or more oligonucleotides, real time PCR, and high resolution melt analysis.

In certain embodiments, the methods as described herein are used to screen the fetus for a genetic disorder.

Certain embodiments of the present disclosure provide a method of pre-natal screening of a fetus, the method comprising amplifying a target DNA sequence from the fetus with one or more target DNA sequence specific primers for at least one or more cycles of amplification of genomic DNA from the fetus with one or more degenerate oligonucleotide primers to produce an amplified target DNA sequence, and assessing the amplified target DNA sequence to screen the fetus.

Certain embodiments of a present disclosure provide a method of pre-natal screening of a fetus, the method comprising:
amplifying genomic DNA from the fetus with one or more degenerate oligonucleotide primers;
for at least one or more cycles of the amplification of the genomic DNA also amplifying a target DNA sequence from the fetus with one or more target DNA sequence specific primers to produce an amplified target DNA sequence; and
assessing the amplified target DNA sequence to screen the fetus.

Certain embodiments of the present disclosure provide a method of pre-natal screening of a fetus, the method comprising:
performing whole genome amplification of the genomic DNA from one or more sperm with one or more degenerate oligonucleotide primers;
for at least part of the whole genome amplification also amplifying a target DNA sequence from the fetus with one or more target DNA sequence specific primers to produce an amplified target DNA sequence; and
assessing the amplified target DNA sequence to screen the fetus.

Certain embodiments of the present disclosure provide a kit for performing a method as described herein.

In certain embodiments, the kit comprises one or more components as described herein, such as one or more of buffers, reagents, enzymes, solutions, dNTPs, controls and/or instructions.

For example, a kit for use for amplifying a target DNA sequence may contain one or more degenerate oligonucleotide primers for whole genome amplification, one or more primers for amplifying a target DNA sequence, and optionally one or more reagents and/or instructions for amplifying the target DNA sequence during whole genome amplification.

Certain embodiments of the present disclosure provide a combination product comprising one or more components as described herein.

In certain embodiments, a combination product is used to perform a method as described herein.

Certain embodiments of the present disclosure provide a combination product, the product comprises the following components:
(i) one or more degenerate oligonucleotide primers for whole genome amplification;
(ii) one or more primers for amplifying a target DNA sequence; and
(iii) one or more reagents and/or instructions for amplifying the target DNA sequence during whole genome amplification.

In certain embodiments, the combination product optionally further comprises one or more other components as described herein, such as one or more of buffers, reagents, enzymes, solutions, dNTPs, controls and/or instructions.

Standard techniques and equipment may be used for recombinant DNA technology, oligonucleotide synthesis, molecular biology and enzymatic reactions. The foregoing techniques and procedures may be generally performed according to methods known in the art and/or as commercially available, and are as described for example in Sambrook *et al.* Molecular Cloning: A Laboratory Manual (4th ed., Cold Spring Harbor Laboratory Press, Cold Spring Harbor, N.Y. (1989)) and Ausubel *et al* Current Protocols in Molecular Biology (2003) John Wiley & Sons, .

The present disclosure is further described by the following examples. It is to be understood that the following description is for the purpose of describing particular embodiments only and is not intended to be limiting with respect to the above description.

### EXAMPLE 1 - Whole genome amplification and gene targeting protocol

The following protocol was used for the whole genome amplification (WGA) and gene targeting studies described herein.

### 1. Materials & Methods

First round whole genome amplification and gene targeting protocol:
(i) Add 1 µl Cell Lysis Enzyme (thermostable protease; EmbryoCellect^{™} kit, RHS; catalogue number RHS1001) to 6.5 µl PCR-grade H₂O; this is called Cell Lysis Enzyme Dil 1.
(ii) Create Cell Lysis Mix by combining the reagents in Table 1:

**Table 1**

| Component | Volume for 1 lysis reactions |
|---|---|
| PCR-grade H₂O | 2.7 |
| Cell Lysis Buffer | 0.15 |
| Cell Lysis Enzyme Dil 1 | 0.15 |
| Total volume | 3 |

(iii) Add 3µl Cell Lysis Mix to the cell or DNA tube. Tap solution to bottom of tube or centrifuge spin briefly. Do not vortex.

In this regard, a single cell typically contains approximately 6 pg DNA.
(iv) Incubate sample in the thermal cycler with the parameters in Table 2:

**Table 2**

| Temperature (°C) | Duration | Number of cycles |
|---|---|---|
| 75°C | 10 min | 1 cycle |
| 95°C | 5 min | 1 cycle |
| 4°C | Hold | 1 cycle |

(v) Prepare a mastermix for the required number of samples as outlined in Table 3, using the commercially reagents listed:

**Table 3**

| Reagent | 1x |
|---|---|
| Ultra-Pure H₂O | 6.5 |
| WGA Buffer (EmbryoCellectTM kit, RHS; catalogue number RHS1001) | 12.5 |
| DOP Primer (EmbryoCellectTM RHS 1001) | 2.5 |
| WGA DNA Polymerase (EmbryoCellectTM kit, RHS; catalogue number RHS1001) | 0.5 |
| lysed single cell (template) | 3.0 |

(vi) Other suitable WGA DNA polymerases are as described herein. For example Cat. # 600675, KAPA HiFi; Kapa Biosystems Cat # KK2101, KAPA2G Robust PCR kits; Kapa Biosystems Cat. # KK5004, Herculase II Fusion DNA Polymerases (Cat # 600675) and AmpliTaq DNA Polymerase ThermoFisher Scientific Cat. # N8080171.
(vii) Aliquot mastermix - 22 µL to PCR tubes containing genomic DNA or lysed cells.
(viii) Run samples through the program in Table 4:

**Table 4**

| Low St (Total ~1:20hr) | | |
|---|---|---|
| Cycles | Temperature | Duration |
| 1 | 95°C | 5min |
| 8 | 98°C | 20 sec |
| | 25°C | 1min 30sec |
| | ramp to 72°C | 1°C/4sec |
| | 72°C | 1min |
| - | 4°C | hold |

(ix) The hold at 4°C is to allow the addition of the gene specific primers to the PCR.
(x) Dilute gene specific primers (Table 8) to required working stock concentrations (10 uM, 5 uM, 2 uM) with PCR grade H₂O.
(xi) During the 4°C hold, while samples are on ice: Add 1.4 uL forward gene specific primer to each tube, add 1.4uL reverse gene specific primer to each tube. Briefly centrifuge the sample tube and place back into the thermocycler.
(xii) Run samples through the program shown in Table 5:

**Table 5**

| High St (Total ~1hr) | | |
|---|---|---|
| Cycles | Temperature | Duration |
| 21 | 98°C | 20 sec |
| | 58°C | 1min |
| | 72°C | 1min |
| 1 | 72°C | 1min |
| | 15°C | soak |

(xiii) Visualise amplification products by running on a 1% agarose gel at 100v for 30 minutes.

Second Round Gene Specific PCR Protocol:
(i) Dilute PCR products generated from the first round PCR 1/50 with PCR-grade water to an approximate concentration of 1 ng/uL.
(ii) Prepare mastermix as outlined in Table 6:

**Table 6**

| Reagent | 1x |
|---|---|
| H₂O | 16.4 |
| 5x KAPA2G Buffer A | 5 |
| MgCl₂ (25mM) | 1 |
| dNTPs (10 mM) | 0.5 |
| Forward Primer (Table 8) | 0.5 |
| Reverse Primer (Table 8) | 0.5 |
| KAPA 2G Polymerase | 0.1 |
| Template | 1.0 |
| mastermix aliquot per tube | 24 µL |
| final volume per tube | 25 µL |

(iii) The KAPA2G Robust kit with dNTPS (Kapa Biosystems, USA, MA; catalogue number KK5004 or KK5005) was utilized to specifically amplify target DNA.
(iv) Aliquot mastermix (24 µL) to each PCR tube.
(v) Add 1 uL of 1/50 diluted template DNA.
(vi) Run samples through the PCR Program shown in Table 7:

**Table 7**

| cycles | temperature | duration |
|---|---|---|
| 1 | 95°C | 3min |
| 25 | 95°C | 30sec |
| | 56°C | 15sec |
| | 72°C | 30sec |
| 1 | 72°C | 1min |
| | 4°C | soak |

(vii) Visualise amplification products by running on a 2% agarose gel at 100v for 30 minutes.

**Table 8. Gene specific primers used:**

| | Primer | Sequence | Melting Temperature | Expected band | GC content |
|---|---|---|---|---|---|
| Forward | D4S43 F-Out | AAATTTTTTGCCAATAAAGATA (SEQ ID NO. 1) | 53.0°C | 283bp | 18% |
| Reverse | D4S43 R-Out | TCTCAGCAAGCTATGAGTAGGT (SEQ ID NO. 2) | 56.1°C | | 45% |
| Forward | Hum-SRY 12fin | CATGAACGCATTCATCGTGTGGTC (SEQ ID NO. 3) | 57.0°C | 254bp | 50% |
| Reverse | Hum-SRY 12rin | CTGCGGGAAGCAAACTGCAATTCTT (SEQ ID NO. 4) | 58.0°C | | 48% |

(viii) Final mastermix concentration of each forward and reverse primer in 25uL solution + 2.8uL additional (Total volume 27.8uL):
(ix) 10uM working stock: 0.5035uM; 5uM working stock: 0.2518uM; 2uM working stock: 0.1007uM.

The DNA sequence of the amplification products may be determined if desired.

One or more of the above reagents may be incorporated in a kit or a combination product, which may also include instructions.

The amplifying of a target DNA sequence as described above has a variety of uses, for example in preimplantation genetic screening, preimplantation genetic diagnosis, prenatal screening, genetic screening, screening for a disease, screening for a cancer, screening for genetic markers, and/or forensic screening.

### EXAMPLE 2 - Validating enrichment of targeted genes in WGA PCR using single cells

Studies were conducted to validate the protocol for enrichment of target genes.

Two sets of gene specific primers (P) (0.5uM, 0.25uM or 0.1uM concentrations) were added to the WGA (multiplex PCR; Fig. 1 and Fig 2) and first round whole genome amplification and gene targeting was performed as described in Example 1 using single cells manually sorted from a commercially available cell line (Coriell; 47,XY,+15 or 48,XXY,+21).

As can be seen, the amplification process resulted in the whole genome amplification of the genomic DNA and the specific amplification of the targeted gene, with amplification of the targeted gene observed at the selected primer concentrations tested. It was noted that WGA amplification in the presence of gene specific primers (P) was slightly weaker than control WGA. These results confirm the ability to amplify a targeted gene using overlapping WGA and gene specific amplification from a single cell.

This data also confirmed that a primer concentration of at least 0.1 µM can be used to amplify a targeted gene from a single cell.

To assess the whole genome amplification, the WGA samples were hybridized to confirm karyotype of the single cell using EmbryoCellectTM array according to the EmbryoCellect^{™} TDS protocol. Correct aneuploidy results were obtained for all single cells tested (Table 9).

**Table 9: EmbryoCellectTM array results for single cell samples following whole genome amplification combined with gene targeted amplification of DNA.**

| **Single Cell Sample** | **WGA Primer** | **Gene Specific Primers** | **Karyotype** | **aCGH Result** | **Array SD** | **Ratio +15** | **Ratio +21** |
|---|---|---|---|---|---|---|---|
| GM04965-45 | Yes | 0.5uM | 48,XXY,+21 | 48,XXY,+21 | 0.06 | - | 1.44 |
| GM04965-46 | Yes | 0.5uM | 48,XXY,+21 | 48,XXY,+21 | 0.07 | - | 1.38 |
| GM04965-41 | Yes | 0.25uM | 48,XXY,+21 | 48,XXY,+21 | 0.08 | - | 1.45 |
| GM04965-42 | Yes | 0.25uM | 48,XXY,+21 | 48,XXY,+21 | 0.07 | - | 1.31 |
| GM04965-43 | Yes | 0.1uM | 48,XXY,+21 | 48,XXY,+21 | 0.05 | - | 1.45 |
| GM04965-44 | Yes | 0.1uM | 48,XXY,+21 | 48,XXY,+21 | 0.05 | - | 1.37 |
| GM04965-103 | Yes | - | 48,XXY,+21 | 48,XXY,+21 | 0.06 | - | 1.31 |
| GM04965-104 | Yes | - | 48,XXY,+21 | 48,XXY,+21 | 0.04 | - | 1.35 |
| GM04965-105 | Yes | - | 48,XXY,+21 | 48,XXY,+21 | 0.06 | - | 1.42 |
| GM04965-106 | Yes | - | 48,XXY,+21 | 48,XXY,+21 | 0.05 | - | 1.34 |
| GM07189-61 | Yes | 0.5uM | 47,XY,+15 | 47,XY,+15 | 0.10 | 1.39 | - |
| GM07189-56 | Yes | 0.25uM | 47,XY,+15 | 47,XY,+15 | 0.05 | 1.31 | - |
| GM07189-58 | Yes | 0.1uM | 47,XY,+15 | 47,XY,+15 | 0.06 | 1.36 | - |
| GM07189-59 | Yes | 0.1uM | 47,XY,+15 | 47,XY,+15 | 0.06 | 1.29 | - |
| GM07189-74 | Yes | - | 47,XY,+15 | 47,XY,+15 | 0.04 | 1.32 | - |
| GM07189-75 | Yes | - | 47,XY,+15 | 47,XY,+15 | 0.07 | 1.26 | - |
| GM07189-76 | Yes | - | 47,XY,+15 | 47,XY,+15 | 0.05 | 1.27 | - |
| GM07189-77 | Yes | - | 47,XY,+15 | 47,XY,+15 | 0.04 | 1.31 | - |

WGA products were then tested for the presence of the targeted genes in gene specific PCRs. Gene enrichment was observed when the bands of the correct size were produced as compared to the absence of the bands in the control (Figure 3 and Figure 4).

These results confirm the ability to amplify a target sequence of interest during a whole genome amplification.

The ability to amplify a target sequence of interest during whole genome amplification provides a number of advantages. For example, the ability to amplify a target sequence provides advantages such as one or more of (i) reducing the possibility of allele drop out when using whole genome amplification, (ii) improved ability to analyse a target DNA sequence amplified from whole genome amplified DNA, (iii) reducing bias associated with amplification of a target DNA sequence amplified from whole genome amplified DNA, (iv) reducing the number of PCR cycles for amplifying a target DNA sequence from genomic DNA, (v) an improvement in the time to amplify a specific target DNA sequence from whole genome amplified DNA, and (vi) reduced use of consumables and/or human resources and/or reduced costs.

### EXAMPLE 3 - Targeted sequence enrichment (TSE) of the HBB gene

Analysis of the targeted sequence enrichment of the HBB gene encoding beta-globin using the protocol was undertaken, for the purposes of further confirming the ability of the protocol to effectively amplify other target sequences, and also for the purposes of assessing the protocol for use in genetic screening and/or diagnosis, such as use of the protocol for pre-implantation genetic diagnosis and pre-implantation genetic screening.

Combined whole genome amplification and HBB gene targeted amplification using two primer sets was performed using multi cell samples (5-cell). The PCR products were then seeded into two specific HBB PCRs; amplifying primarily exon 1 and 2, and amplifying exon 3. The second round HBB PCR amplicons were both pooled to generate a final dilution of 1:20 with the whole genome amplified DNA with HBB gene targeted amplification. The WGA only PCR products, the combined whole genome amplification and HBB gene targeted amplification products and the 1:20 diluted pooled DNA were sequenced as individually indexed samples in a 40 sample multiplex MiSeq run; 2x75bp read length. Next generation sequencing data was bioinformatically aligned to hg19. Breadth and depth of HBB was calculated for each of the samples and compared. Mean depth of coverage of > 60x was achieved when the 1:20 diluted pooled DNA was sequenced.

Figure 5 shows the breath of coverage of HBB following combined whole genome amplification and gene targeted amplification compared to whole genome amplification only using next generation sequencing. After combined whole genome amplification and gene targeted amplification products were seeded into two gene specific HBB PCRs. The second round PCR amplicons were pooled to generate a final dilution of 1:20 with the whole genome amplified DNA with gene targeted amplification. The combined whole genome amplification and gene targeted amplification products (WGA + TSE) and the 1:20 diluted pool were sequenced. Next generation sequencing data was bioinformatically aligned to hg19 and the coverage was determined at a depth of 1x for each of the samples. The graph shows 100% breadth of coverage of HBB was achieved when the 1:20 dilution of second round HBB PCR gene specific products were pooled back with the combined whole genome amplified samples with gene targeted amplification. Mean values plotted.

Figure 6 shows an analysis of the depth of coverage for the HBB gene. Mean depth of coverage of HBB > 60x was achieved when the 1:20 diluted pooled DNA was sequenced. This is a depth suitable for genetic analysis for SNP determination.

Combined whole genome amplification and sequence specific amplification can be used to achieve 100% breadth of coverage of target DNA sequence and increase the depth of coverage suitable for genetic analysis for SNP detection.

In addition, we have also demonstrated in related experiments the successful multiplexing with up to 4 primer sets.

### EXAMPLE 4 - Targeted sequence enrichment of the gene for Major Histocompatibility Complex, Class I, A

Analysis of the targeted sequence enrichment of the gene encoding the Major Histocompatibility Complex, Class I, A protein using the protocol was undertaken, for the purposes of further assessing the protocol to effectively amplify other target sequences and also for the purposes of further assessing the protocol for use in genetic screening and/or diagnosis, such as pre-implantation genetic diagnosis and pre-implantation genetic screening.

Combined whole genome amplification and human leukocyte antigen (HLA) gene targeted amplification was performed using multi cell samples (5-cell). The PCR products were then seeded into one gene specific HLA PCR. The second round HLA PCR amplicons were pooled to generate a final dilution of 1:20 and 1:10 with the whole genome amplified DNA with HLA gene targeted amplification. The WGA only PCR products, the combined whole genome amplification and HLA gene targeted amplification products, the 1:20 and 1:10 diluted pooled DNA and the second round HLA PCR amplicon were sequenced as individually indexed samples in a 40 sample multiplex MiSeq run; 2x75bp read length. Next generation sequencing data was bioinformatically aligned to hg19. Breadth and depth of HLA was calculated for each of the samples and compared. Mean depth of coverage of HLA > 60x was achieved when the 1:20 and 1:10 diluted pooled DNA and the second round PCR amplicon were sequenced.

Figure 7 shows the breadth of coverage for HLA following combined whole genome amplification and gene targeted amplification compared to whole genome amplification only using next generation sequencing. After combined whole genome amplification and gene targeted amplification products were seeded into one HLA specific PCR. The second round PCR amplicons were pooled to generate a final dilution of 1:20 and 1:10 with the whole genome amplified DNA with gene targeted amplification. The WGA only PCR products, the combined whole genome amplification and gene targeted amplification products, the 1:20 and 1:10 diluted pooled DNA and the second round PCR amplicon were sequenced. Next generation sequencing data was bioinformatically aligned to hg19 and the coverage of exon 2 and exon 3 was determined at a depth of 1x for each of the samples. Mean depth of coverage of > 60x was achieved when the 1:20 and 1:10 diluted pooled DNA and the second round PCR amplicon were sequenced.

Figure 8 shows an analysis of the depth of coverage for the HLA gene.

Mean depth of coverage of > 60x was achieved when the 1:20 and 1:10 diluted pooled DNA and the second round HLA PCR amplicon were sequenced. This is a depth suitable for genetic analysis for SNP detection.

### EXAMPLE 5 - PGS of Embryos

One use of the target gene enrichment protocol described herein is for pre-implantation genetic screening of an embryo prior to transfer into the uterus.

The screening requires the removal of one to several cells from the embryo by biopsy. Methods for removing cells from an embryo are known in the art. These cell(s) are then placed into a tube and due to the limited amount of DNA in the biopsy material, the DNA is required to be amplified to generate enough DNA for down-stream technology.

The targeted sequence enrichment protocol can then be used to determine the chromosome complement of the embryo. In addition, the targeted sequence enrichment protocol can be used for preimplantation genetic screening of the embryo of specific genes and/or other loci of interest.

### EXAMPLE 6 - Generalised protocol, kits & products

A generalised protocol, utilising various kits, components and instructions, for conducting targeted sequence enrichment is as follows:

### 1. Kit Contents

A kit for conducting targeted sequence enrichment may contain the following components:
- PCR-grade H₂0
- Cell Lysis Enzyme
- Cell Lysis Buffer
- Polymerase
- PCR Buffer
- DOP Primer

### 2. Overview of Protocol

Degenerate Oligonucleotide Primed PCR (DOP-PCR)-based Whole Genome Amplification (WGA) generates representative amplification of total DNA from single cells or their DNA equivalent. The DOP-PCR-based WGA reproducibly amplifies total DNA from single cells to produce microgram quantities of amplified DNA in less than 3 hours. The protocol can be used successfully on both cellular and purified genomic DNA inputs.

The protocol involves the following general steps:
(i) Sample collection
   - Place the sample into a PCR tube in <2 µl of buffer
   - Mark the sample location on the tube
(ii) Lysis
   - Add 3 µl of lysis solution above the sample
   - Tap the PCR tube to allow the lysis solution to roll over the sample
   - Incubate for 15 min according to the lysis program

It will be appreciated that under some circumstances the cell lysis step may be omitted, permitting, for example, use of the protocol on cell-free DNA, or that the protocol may achieved by directly entering the amplification protocol using a cell(s) and not having a discrete lysis step prior to the amplification.

(iii) Whole Genome Amplification and Targeted Sequence Enrichment
- Add 22 µl of PCR master mix to the lysed sample
- PCR using the WGA PCR program and sequence specific primers
- Assess WGA by agarose gel electrophoresis

### 3. Input Specifications

### (i) Number of Cells

The protocol described here is suitable for single cells or its DNA equivalent, as well as small numbers of cells (e.g. <10 cells).

### (ii) Cell Collection Method

Flow sorting, dilution and micromanipulation are collection methods compatible with the protocol. Single cells should be transferred to a PCR tube with minimal transfer buffer (<2 µl). The location of the cell in the tube should be marked with a dot on the outside of the tube using a permanent marker pen so as to enable easy cell location for the lysis step.

### (iii) DNA Dilution

It is recommended that DNA is diluted to a final concentration of 30 pg/µl in 10mM Tris-HCl (pH 8.0) (No EDTA).

### (iv) Compatible Buffers

Recommended cell transfer buffers include 10 mM Tris-HCl (pH 8.0) (No EDTA) and PBS (Mg²⁺, Ca²⁺ free and BSA free).

### 4. Protocols: DOP-PCR Whole Genome Amplification

### (i) Cell Lysis

In this optional step, cells are lysed and DNA is made soluble with the addition of a Cell Lysis Mix and a short incubation in a PCR thermocycler.

Consumables:
- 0.2mL or 0.5mL sterile PCR tubes
- PCR-grade H₂O
- Cell Lysis Enzyme
- Cell Lysis Buffer
- Cell or DNA samples

Preparation:
- Remove PCR-grade H₂O and Cell Lysis Buffer from storage and thaw to room temperature.
- Remove Cell Lysis Enzyme from storage and store in a cold block at 4°C.
- Mix reagents well then briefly centrifuge to collect contents at the bottom of the tube.
- Calculate volumes of reagents required for the Cell Lysis Mix.

### Procedure:

Prepare Cell Lysis Enzyme Dilution 1 in a 4°C cold block by combining the following reagents:

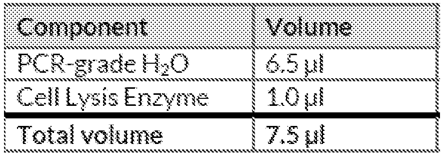

### Mix well then briefly centrifuge

Prepare Cell Lysis Mix in a 4°C cold block for the required number of reactions by combining the following reagents:

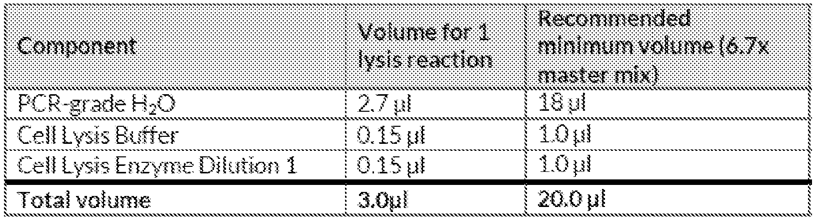

Steps for Lysing Single Cells/Multi Cell samples:
(a) Add 3 µl of Cell Lysis Mix above the cell sample located in a PCR tube. Make sure that the lysis mix rolls over the sample location as marked on the tube by gently tapping the tube on the benchtop. Do not mix or vortex.
(b) Briefly spin in a mini centrifuge if required to collect contents at the bottom of the tube.
(c) Repeat with other samples.

Steps for No Template Control (NTC) Preparation:
(a) Add 3 µl of Cell Lysis Mix to 1 sterile PCR tube labelled NTC.
(b) Add 1 µl of PCR-grade H2O to the tube labelled NTC.

Steps for DNA Sample Preparation prior to WGA PCR (if required):
(a) Add 3 µl of Cell Lysis Mix to the required number of sterile empty PCR tubes.
(b) Add 1µl of 30 pg/µl DNA sample to each tube containing Cell Lysis Mix. Proceed to next step.

Incubate all samples and NTC in a thermocycler programmed as follows:

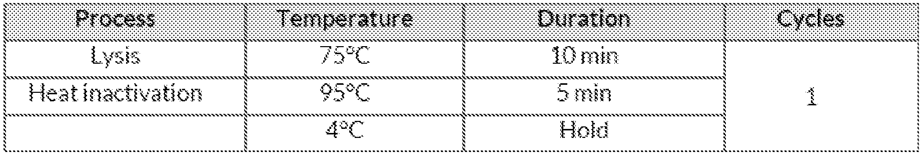

Place the lysed samples in a cold block.

### (ii) Whole Genome Amplification and Targeted Sequence Enrichment

In this step a master mix is created and added to the lysed samples before Degenerate Oligonucleotide Prime PCR (DOP-PCR) based Whole Genome Amplification (WGA). This generates representative amplification of total DNA from cells or their DNA equivalent.

Consumables:
- 1.5mL tube
- PCR-grade H₂O
- WGA PCR Buffer
- DOP Primer
- WGA Polymerase
- Lysed samples and NTC (from Cell Lysis)
- Sequence Specific Primers

Use of a variety of different DOP primers is possible. Examples of DOP primers include DOP primers which have a part of their nucleotide sequence that allows the possibility of more than one base at a particular position, and DOP primers that can form base pairs with one or more other bases may also be used.

Preparation:
- Dilute Sequence Specific Primers to required concentration in user supplied PCR-grade H₂O.
- Remove PCR-grade H₂O, WGA PCR Buffer and Primer from storage and thaw to room temperature.
- Remove WGA Polymerase from storage and store in a cold block at 4°C.
- Mix reagents well then briefly centrifuge to collect contents at the bottom of the tube.
- Calculate volumes of reagents required for the WGA master mix.

Procedure:
- Prepare WGA master mix for the required number of reactions by combining the following reagents in a 1.5mL tube in the order they are listed below: 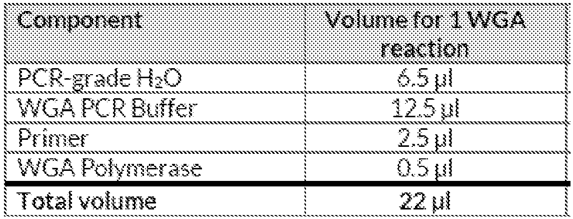
- Mix well then briefly spin down in a mini centrifuge.
- Transfer 22 µl of PCR master mix to individual tubes containing lysed template (sample or NTC in Cell Lysis Mix). To prevent removal of any DNA from the sample, do not insert the pipette tip into lysed sample mix. Do not mix or vortex the PCR tubes. Briefly centrifuge or spin in mini centrifuge to collect contents at the bottom of the tube.
- Transfer 22 µl of PCR master mix to the individual tubes containing DNA template in Cell Lysis Mix. To prevent removal of any DNA from the sample, do not insert the pipette tip into lysed sample mix. Mix well then briefly centrifuge to collect contents at the bottom of the tube.
- Incubate all samples and NTC in a thermocycler programmed as follows:

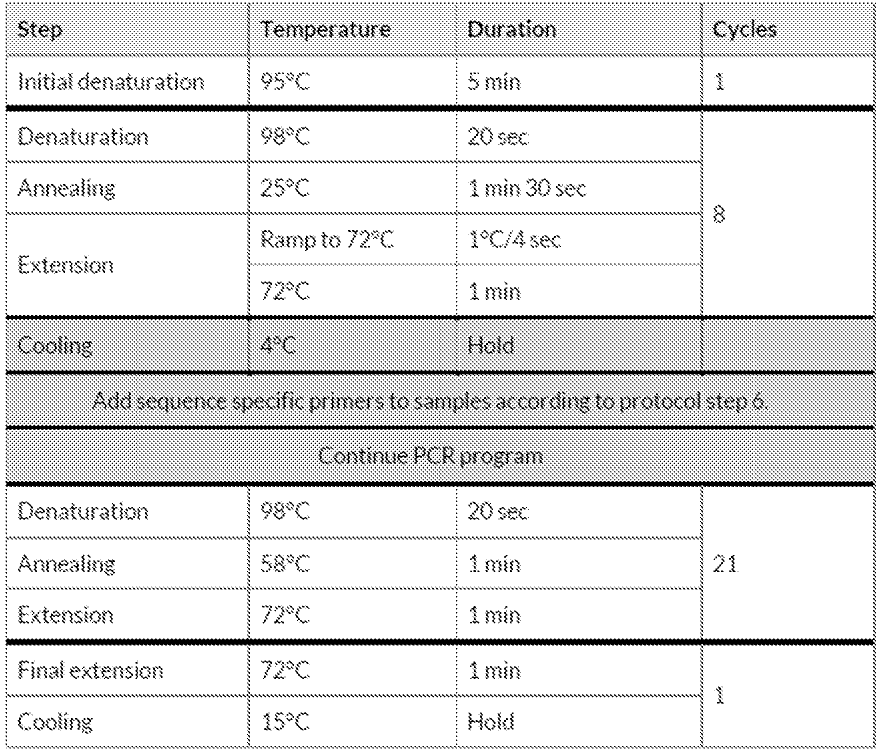

### (iii) Steps for Diluting Sequence Specific Primers prior to WGA with TSE PCR

Procedure:
- It is recommended that each primer set is optimised for use in the WGA PCR using a 30pg gDNA template.
- Dilute sequence specific primers to the required working stock concentration with PCR-grade H₂O.
- As a guide, titrate the primer concentration using working stock concentrations of 2uM, 5uM and 10uM. Confirm sequence enrichment using semi-quantitative PCR (low number of PCR cycles, where the targeted band is just visible on a gel. Do not run the PCR to plateau phase) or an alternate method, to finalise the optimal working stock primer concentration.
- As an example, nested PCR directed towards the target regions can be used to confirm that the targeted sequence enrichment has occurred.
- Confirmation that the optimal primer concentration has been used is also provided by running the WGA products on an agarose gel to ensure the PCR amplicons are the typical size and quantity for DOPlifyTM WGA.
- At the HOLD step during the PCR program, transfer 1.4 µl of 5' (forward) primer and 1.4 µl 3' (reverse) primer to each tube containing the DNA template in WGA mix. The volume of all primers in a multiplexed set added to the sample should not exceed 2.8 µl in total. To prevent removal of any sample DNA, do not insert the pipette tip into the sample mix. Instead, pipette the primers on to the side of the PCR tube, just above the sample master mix level.
- Briefly centrifuge to collect contents at the bottom of the tube.
- Resume the WGA PCR program.
- On completion of the PCR, store the DNA either at 4°C short term or -20°C long term.
- Quality of WGA DNA may be assessed, for example by gel electrophoresis. WGA DNA products should appear as a smear of products, ranging from 200 bp - 2000 bp. NTC should appear clean, with the presence of primer dimers.
- A failed WGA amplification is indicated by the presence of primer dimers, but no evidence of the larger amplification products.
- Poor WGA amplification is indicated by smears with lower intensity or with PCR products that are notably larger or smaller than the expected size range observed for the other samples on the same agarose gel.
- Confirmation of targeted sequence enrichment using sequence specific PCR and whole genome amplified DNA as the PCR template. An agarose gel will show enriched amplification of the target sequence due to the addition of gene specific primers to the WGA PCR compared with the control.

Also, it is to be noted that, as used herein, the singular forms "a", "an" and "the" include plural aspects unless the context already dictates otherwise.

Throughout this specification, unless the context requires otherwise, the word "comprise", or variations such as "comprises" or "comprising", will be understood to imply the inclusion of a stated element or integer or group of elements or integers but not the exclusion of any other element or integer or group of elements or integers.

Reference to any prior art in this specification is not, and should not be taken as, an acknowledgment or any form of suggestion that this prior art forms part of the common general knowledge in any country.

All methods described herein can be performed in any suitable order unless indicated otherwise herein or clearly contradicted by context.

## Claims

1. A method of amplifying a target DNA sequence from genomic DNA, the method comprising amplifying the genomic DNA by polymerase chain reaction with one or more degenerate oligonucleotide primers, and subsequently amplifying the amplified genomic DNA with the one or more degenerate oligonucleotide primers in the presence of one or more target DNA sequence specific primers, wherein the amplifying with the one or more degenerate oligonucleotide primers comprises amplifying with the one or more degenerate oligonucleotide primers for 1 to 8 cycles with an annealing temperature of 20°C to 35°C, and the subsequent amplifying comprises amplifying for 5 to 25 cycles with an annealing temperature of 52°C to 65°C, thereby amplifying the target DNA sequence.

2. The method according to claim 1, wherein the one or more degenerate oligonucleotide primers comprise a nucleotide sequence comprising 6 to 12 contiguous random nucleotides.

3. The method according to claims 1 or 2, wherein the method comprises a concentration of the one or more degenerate oligonucleotide primers of 0.1 to 4 µM.

4. The method according to any one of claims 1 to 3, wherein the method comprises a concentration of the one or more target DNA sequence specific primers of 0.05 to 4 µM.

5. The method according to any one of claims 1 to 4, wherein the one or more target DNA sequence specific primers comprise a melting temperature of 50° to 64°C and a GC content of 18% to 65%.

6. The method according to any one of claims 1 to 5, wherein the method comprises an amount of genomic DNA to be amplified of 1 pg to 6 ng.

7. The method according to any one of claims 1 to 6, wherein the method comprises one or more further rounds of amplifying the amplified target DNA sequence with further target DNA sequence specific primers.

8. The method according to any one of claims 1 to 7, wherein the genomic DNA comprises DNA from one or more embryonic cells, DNA from an oocyte or a polar body thereof, DNA from sperm, DNA from one or more germ cells, DNA from one or more somatic cells, DNA from one or more human or animal cells, DNA from one or more plant cells, DNA from one or more cells from a microorganism, DNA from one or more cells for screening for a disease, DNA from one or more cancerous cells, DNA for forensic testing, mitochondrial DNA, extra chromosomal DNA, cell-free DNA, DNA from a sample comprising one or more cells, DNA from a sample comprising cell-free DNA, DNA from a cell lysate, DNA from a biopsy, DNA from a tissue sample, DNA from a cell sample, DNA from a bodily fluid, DNA from a blood sample, DNA from amniotic fluid, DNA from a formalin fixed sample, DNA from a paraffin embedded sample, and DNA from a swab.

9. The method according to any one of claims 1 to 8, wherein the genomic DNA comprises DNA from a single cell.

10. Use of a kit for performing a method according to any one of claims 1 to 9, the kit comprising one or more degenerate oligonucleotide primers, one or more primers for amplifying a target DNA sequence, and optionally one or more reagents and/or instructions for amplifying the target DNA sequence during whole genome amplification.

## Patentansprüche

1. Verfahren zum Amplifizieren einer Ziel-DNA-Sequenz von genomischer DNA, das Verfahren umfassend Amplifizieren der genomischen DNA durch Polymerasekettenreaktion mit einem oder mehreren degenerierten Oligonukleotid-Primern, und anschließend Amplifizieren der amplifizierten genomischen DNA mit dem einen oder den mehreren degenerierten Oligonukleotid-Primern in Anwesenheit von einem oder mehreren Ziel-DNA-Sequenz-spezifischen Primern, wobei das Amplifizieren mit dem einen oder den mehreren degenerierten Oligonukleotid-Primern Amplifizieren mit dem einen oder den mehreren degenerierten Oligonukleotid-Primern für 1 bis 8 Zyklen mit einer Annealing-Temperatur von 20 °C bis 35 °C umfasst, und das anschließende Amplifizieren Amplifizieren für 5 bis 25 Zyklen mit einer Annealing-Temperatur von 52 °C bis 65 °C umfasst, wodurch die Ziel-DNA-Sequenz amplifiziert wird.

2. Verfahren nach Anspruch 1, wobei der eine oder die mehreren degenerierten Oligonukleotid-Primer eine Nukleotidsequenz umfassen, die 6 bis 12 zusammenhängende zufällige Nukleotide umfasst.

3. Verfahren nach Ansprüchen 1 oder 2, wobei das Verfahren eine Konzentration des einen oder der mehreren degenerierten Oligonukleotid-Primer von 0,1 bis 4 µM umfasst.

4. Verfahren nach einem der Ansprüche 1 bis 3, wobei das Verfahren eine Konzentration des einen oder der mehreren Ziel-DNA-Sequenz-spezifischen Primer von 0,05 bis 4 µM umfasst.

5. Verfahren nach einem der Ansprüche 1 bis 4, wobei der eine oder die mehreren Ziel-DNA-Sequenz-spezifischen Primer eine Schmelztemperatur von 50 ° bis 64 °C und einen GC-Gehalt von 18 % bis 65 % umfassen.

6. Verfahren nach einem der Ansprüche 1 bis 5, wobei das Verfahren eine Menge an genomischer DNA, die zu amplifizieren ist, von 1 pg bis 6 ng umfasst.

7. Verfahren nach einem der Ansprüche 1 bis 6, wobei das Verfahren eine oder mehrere weitere Runden des Amplifizierens der amplifizierten Ziel-DNA-Sequenz mit weiteren Ziel-DNA-Sequenz-spezifischen Primern umfasst.

8. Verfahren nach einem der Ansprüche 1 bis 7, wobei die genomische DNA DNA aus einer oder mehreren embryonalen Zellen, DNA aus einer Eizelle oder einem Polkörperchen davon, DNA aus Sperma, DNA aus einer oder mehreren Keimzellen, DNA aus einer oder mehreren somatischen Zellen, DNA aus einer oder mehreren menschlichen oder tierischen Zellen, DNA aus einer oder mehreren Pflanzenzellen, DNA aus einer oder mehreren Zellen aus einem Mikroorganismus, DNA aus einer oder mehreren Zellen zum Screenen auf eine Erkrankung, DNA aus einer oder mehreren kanzerösen Zellen, DNA für forensische Prüfung, mitochondriale DNA, extrachromosomale DNA, zellfreie DNA, DNA aus einer eine oder mehrere Zellen umfassenden Probe, DNA aus einer zellfreie DNA umfassenden Probe, DNA aus einem Zelllysat, DNA aus einer Biopsie, DNA aus einer Gewebeprobe, DNA aus einer Zellprobe, DNA aus einer Körperflüssigkeit, DNA aus einer Blutprobe, DNA aus Fruchtwasser, DNA aus einer Formalin-fixierten Probe, DNA aus einer Paraffin-eingebetteten Probe und DNA aus einem Abstrich umfasst.

9. Verfahren nach einem der Ansprüche 1 bis 8, wobei die genomische DNA DNA aus einer einzigen Zelle umfasst.

10. Verwendung eines Kits zum Durchführen eines Verfahrens nach einem der Ansprüche 1 bis 9, wobei das Kit einen oder mehrere degenerierte Oligonukleotid-Primer, einen oder mehrere Primer zum Amplifizieren einer Ziel-DNA-Sequenz und wahlweise ein oder mehrere Reagenzien und/oder Anleitungen zum Amplifizieren der Ziel-DNA-Sequenz während Gesamtgenomamplifikation umfasst.

## Revendications

1. Procédé d'amplification d'une séquence d'ADN cible à partir d'ADN génomique, le procédé comprenant l'amplification de l'ADN génomique par une réaction en chaîne de la polymérase avec une ou plusieurs amorces oligonucléotidiques dégénérées et par la suite l'amplification de l'ADN génomique amplifié avec les une ou plusieurs amorces oligonucléotidiques dégénérées en présence d'une ou de plusieurs amorces spécifiques à la séquence d'ADN cible, dans lequel l'amplification avec les une ou plusieurs amorces oligonucléotidiques dégénérées comprend l'amplification avec les une ou plusieurs amorces oligonucléotidiques dégénérées pendant 1 à 8 cycles avec une température d'annelage de 20 °C à 35 °C, et l'amplification suivante comprend une amplification pendant 5 à 25 cycles avec une température d'annelage de 52 °C à 65 °C, amplifiant ainsi la séquence d'ADN cible.

2. Procédé selon la revendication 1, dans lequel les une ou plusieurs amorces oligonucléotidiques dégénérées comprennent une séquence de nucléotides comprenant 6 à 12 nucléotides aléatoires contigus.

3. Procédé selon les revendications 1 ou 2, où le procédé comprend une concentration des une ou plusieurs amorces oligonucléotidiques dégénérées de 0,1 à 4 µM.

4. Procédé selon l'une quelconque des revendications 1 à 3, où le procédé comprend une concentration des une ou plusieurs amorces spécifiques à la séquence d'ADN cible de 0,05 à 4 µM.

5. Procédé selon l'une quelconque des revendications 1 à 4, dans lequel les une ou plusieurs amorces spécifiques à la séquence d'ADN cible comprennent une température de fusion de 50 ° à 64 °C et un taux de GC de 18 % à 65 %.

6. Procédé selon l'une quelconque des revendications 1 à 5, où le procédé comprend une quantité d'ADN génomique à amplifier de 1 pg à 6 ng.

7. Procédé selon l'une quelconque des revendications 1 à 6, où le procédé comprend un ou plusieurs tours d'amplification supplémentaires de la séquence d'ADN cible amplifiée avec des amorces spécifiques à la séquence d'ADN cible supplémentaires.

8. Procédé selon l'une quelconque des revendications 1 à 7, dans lequel l'ADN génomique comprend de l'ADN provenant d'une ou de plusieurs cellules embryonnaires, de l'ADN provenant d'un ovocyte ou d'un corps polaire de celui-ci, de l'ADN provenant de sperme, de l'ADN provenant d'une ou de plusieurs cellules germinales, de l'ADN provenant d'une ou de plusieurs cellules somatiques, de l'ADN provenant d'une ou de plusieurs cellules humaines ou animales, de l'ADN provenant d'une ou de plusieurs cellules végétales, de l'ADN provenant d'une ou de plusieurs cellules issues d'un microorganisme, de l'ADN provenant d'une ou de plusieurs cellules pour le dépistage d'une maladie, de l'ADN provenant d'une ou de plusieurs cellules cancéreuses, de l'ADN pour un test de légiste, de l'ADN mitochondrial, de l'ADN extra chromosomique, de l'ADN acellulaire, de l'ADN provenant d'un échantillon comprenant une ou plusieurs cellules, de l'ADN provenant d'un échantillon comprenant un ADN acellulaire, de l'ADN provenant d'un lysat cellulaire, de l'ADN provenant d'une biopsie, de l'ADN provenant d'un échantillon tissulaire, de l'ADN provenant d'un échantillon cellulaire, de l'ADN provenant d'un fluide corporel, de l'ADN provenant d'un échantillon sanguin, de l'ADN provenant d'un fluide amniotique, de l'ADN provenant d'un échantillon fixé sur de la formaline, de l'ADN provenant d'un échantillon englobé de paraffine et de l'ADN provenant d'un écouvillon.

9. Procédé selon l'une quelconque des revendications 1 à 8, dans lequel l'ADN génomique comprend de l'ADN provenant d'une seule cellule.

10. Utilisation d'un kit pour effectuer un procédé selon l'une quelconque des revendications 1 à 9, le kit comprenant une ou plusieurs amorces oligonucléotidiques dégénérées, une ou plusieurs amorces pour l'amplification d'une séquence d'ADN cible et optionnellement un ou plusieurs réactifs et/ou instructions pour l'amplification de la séquence d'ADN cible pendant l'amplification du génome entier.
